# EUROPÄISCHE PATENTSCHRIFT

(11) **EP 3 930 793 B1**
(45) Veröffentlichungstag und Bekanntmachung des Hinweises auf die Patenterteilung: **08.11.2023**
(21) Anmeldenummer: 21715160.4
(22) Anmeldetag: 22.03.2021
(51) Int. Cl.: A61M 5/20, A61M 5/315, A61M 5/31, A61M 5/32

(54) **AUTOINJEKTOR MIT AUSSCHÜTTDETEKTION**
AUTOINJECTOR WITH DISCHARGE DETECTION
AUTO-INJECTEUR À DÉTECTION DE DISTRIBUTION

(30) Priorität: 26.03.2020 CH 3622020
(43) Veröffentlichungstag der Anmeldung: 05.01.2022
(60) Teilanmeldung: 23199294.2
(73) Patentinhaber: Ypsomed AG, 3401 Burgdorf (CH)
(72) Erfinder: KALBERMATTER, Gabriel, 3400 Burgdorf (CH); URBANEK, Leos, 3012 Bern (CH); TSCHIRREN, Markus, 3400 Burgdorf (CH); HIRSCHEL, Jürg, 3007 Bern (CH); BRÜGGER, Martin, 3065 Bolligen (CH)
(74) Vertreter: Meier Obertüfer, Jürg
(86) Internationale Anmeldenummer: PCT/EP2021/057168
(87) Internationale Veröffentlichungsnummer: WO 2021/191095

(56) Entgegenhaltungen:
- EP-A1- 3 178 507
- WO-A1-02/064196
- WO-A1-02/064196
- WO-A1-2006/069455
- WO-A1-2006/069455
- WO-A1-2006/069455
- WO-A1-2008/037801
- WO-A1-2008/037801
- WO-A1-2008/037801
- WO-A1-2014/139916
- WO-A1-2014/139916
- WO-A1-2017/089259
- WO-A1-2017/089259
- WO-A1-2017/114768
- WO-A1-2017/114768
- WO-A1-2017/114768
- WO-A1-2017/114909
- WO-A1-2017/114909
- WO-A1-2019/036576
- WO-A1-2019/036576
- WO-A1-2019/121612
- WO-A1-2019/121612
- WO-A2-2010/091522
- WO-A2-2010/091522
- CN-A- 102 065 933
- CN-A- 102 065 933

## Beschreibung

### TECHNISCHES GEBIET

Die vorliegende Erfindung betrifft das Gebiet der medizinischen Injektionsgeräte zur Verabreichung von flüssigen Substanzen, insbesondere von Medikamenten oder medizinischen Substanzen wie Insulin- und Hormonpräparationen. Die Erfindung bezieht sich auf einen Autoinjektor mit einem Energiespeicher zur Ausschüttung einer vorgegebenen Dosis aus einem einmalig genutzten Produktbehälter.

### HINTERGRUND DER ERFINDUNG

Injektionsgeräte oder Injektionsvorrichtungen zum vereinfachten Verabreichen einer Substanz umfassen unter anderem so genannte Autoinjektoren, welche einen Energiespeicher aufweisen, mit welchem die Ausschüttung automatisch, das heisst ohne extern von einem Benutzer zuzuführende oder aufzuwendende Kraft, durchgeführt werden kann. Der Energiespeicher speichert die für eine automatische Substanzabgabe erforderliche Energie vorteilhaft in mechanischer Form. Ein solcher Energiespeicher kann eine Feder sein, welche in einem gespannten Zustand in das Injektionsgerät eingebaut wird und durch Entspannen Energie abgibt. Die Energieabgabe erfolgt an eine Kolbenstange oder ein Druckelement, welches einen Kolben in einen Produktbehälter einschiebt. Der Energiespeicher kann auch vorgesehen sein um den Vorgang des Einstechens einer Injektionsnadel zu automatisieren. Alternativ kann der Einstechvorgang manuell erfolgen, also ausschliesslich durch einen Benutzer, ohne hierfür in dem Injektionsgerät gespeicherte Energie zu verwenden.

Das Injektionsgerät kann einen Produktbehälterhalter zur Aufnahme eines Produktbehälters umfassen, wobei in dem Produktbehälterhalter der Produktbehälter radial, axial und vorzugsweise auch drehfest gehalten werden kann. Der Produktbehälterhalter kann mit dem Gehäuse der Injektionsvorrichtung axial- und drehfest verbunden sein, oder bei einem Einstech- und/oder Nadelrückzugsvorgang relativ zum Gehäuse bewegbar sein. Der Produktbehälter kann eine Karpule zur einmaligen oder wiederholt lösbaren Verbindung mit Einweg-Injektionsnadeln oder eine Einweg-Fertigspritze mit einer damit unlösbar verbundenen Injektionsnadel sein. Der Produktbehälter weist einen hohlzylindrischen Produktbehälterabschnitt auf, der einen Kolben oder Stopfen verschiebbar lagert. Der Kolben kann mit dem Innenumfang des Produktbehälterabschnitts einen Dichtspalt bilden und mittels Kolbenstange in eine distale Richtung verschoben werden, um über die Injektionsnadel Produkt aus dem Produktbehälter abzugeben.

Das Injektionsgerät kann eine Nadelschutzhülse aufweisen, die nach erfolgter Injektion distal über das distale Ende der Injektionsnadel steht oder relativ zu dem Gehäuse unter Entspannung einer Nadelschutzhülsenfeder in diese Position verschoben wird, um den versehentlichen Zugriff auf die Injektionsnadel zu verhindern und dadurch ein Verletzungsrisiko zu verringern. Bei einem Autoinjektor kann die Nadelschutzhülse auch als Auslöseelement zum Auslösen der Produktausschüttung dienen, wobei die Nadelschutzhülse hierzu relativ zu dem Gehäuse in die proximale Richtung verschoben wird. Alternativ kann die Auslösung des Autoinjektors durch Betätigen eines Auslöseknopfs des Autoinjektors erreicht werden, wobei die Nadelschutzhülse vor dem Gebrauch des Autoinjektors zumindest als Sichtschutz dient.

Die Patentanmeldung WO 2016/205963 beschreibt einen beispielhaften Autoinjektor, umfassend ein Gehäuse mit Längsachse, eine Auslösevorrichtung, einen axial fest im Gehäuse angeordneten Produktbehälter. Der Autoinjektor umfasst weiter eine in einer Längsrichtung zwischen einer proximalen und einer distalen Position verschiebbare Nadelschutzhülse, welche mit einer Nadelschutzfeder gekoppelt ist. Eine Rückkopplungsvorrichtung mit einem durch die Nadelschutzfeder zu einem Anschlag hin beschleunigten Anschlagelement dient zur Erzeugung eines akustischen Signals nach Abgabe einer bestimmten Menge an Substanz. Eine Spiral- oder Triebfeder, in welcher Energie für das automatische Ausschütten von Produkt gespeichert werden kann, ist mit der Auslösevorrichtung gekoppelt, wobei ein erstes Ende der Spiralfeder mit dem Gehäuse verbunden ist, und ein zweites Ende der Spiralfeder rotationsfest mit einem koaxial zur Längsachse angeordneten Antriebselement in Form einer rotierenden Gewindestange verbunden ist. Die Gewindestange greift über ein Gewinde in ein im Gehäuse nicht rotierendes Vortriebsglied in Form einer hülsenförmigen Kolbenstange, welche bei einer Verschiebung in distale Richtung den Stopfen des Produktbehälters mit einer zumindest annähernd konstanten Ausschüttgeschwindigkeit mitbewegt.

Die Patentanmeldung WO 2017/097507 zeigt einen Autoinjektor mit einer vorgespannten Spiralfeder, einem Antriebselement und einer Kolbenstange, welche durch das mit der Spiralfeder verbundene Antriebselement in Rotation versetzt wird und dadurch einen Kolben in einem Spritzenkörper axial verschiebt. Die Rotation eines Signalisierungselements bei Beginn einer Ausschüttung wird durch Schliessen eines elektrischen Kontakts mit zwei Kontaktpunkten, entweder über eine Bewegung eines die Kontaktpunkte elektrisch isolierenden Streifens oder über eine Relativbewegung der Kontaktpunkte eines mechanischen oder magnetischen Schalters, detektiert, dadurch wird eine Sensoreinheit des Autoinjektors aktiviert. Letztere ermittelt Informationen zur Ausschüttung, beispielsweise eine Anzahl von Umdrehungen des Signalisierungselements, welche anschliessend mit einer vorbekannten Anzahl Umdrehungen verglichen werden kann.

Die Patentanmeldung WO 2019/129622 beschreibt einen manuell betriebenen InjektionsPen mit Dosiswahl, umfassend eine Antriebshülse, welche rotationsfest und axial verschiebbar an eine Kolbenstange koppelt und durch eine Torsionsfeder in Rotation versetzt wird. Ein optischer Rotationssensor detektiert die Rotation der Antriebshülse indem ein in einem Prisma umgelenkter Lichtstrahl von einer Innenseite der Antriebshülse selektiv radial reflektiert wird. Aus der Anzahl registrierter Reflexionen kann auf eine ausgeschüttete Dosis geschlossen werden.

Die Patentanmeldung WO 2019/209491 beschreibt ebenfalls einen manuellen InjektionsPen mit variabler Dosis, umfassend einen kapazitiven, piezoelektrischen, oder resistiven Dehnungssensor zur Erfassung einer Dehnung eines Biegebalkens in Form eines Arms einer Leiterplatine. Diese Dehnung wird durch die Rotationsbewegung eines Zahnrads induziert. Jeder einzelne Zahn des Zahnrads drückt ein Ende des axial ausgerichteten Arms radial nach aussen, so dass die dadurch entstehende mechanische Dehnung detektiert werden kann.

Die Patentanmeldung WO 2017/129314 beschreibt einen Autoinjektor mit einem Detektor zur Detektion eines Starts einer Ausschüttung basierend auf einer Bewegung der Nadelschutzhülse in proximale Richtung. Durch die erfolgte Detektion wird eine Kommunikationseinheit des Autoinjektors aktiviert.

Die Patentanmeldung WO 2018/069150 zeigt einen Autoinjektor mit einem Kontroller zur Detektion des Endes eines Ausschütthubs basierend auf der Induktivität einer leitfähigen Wendel- oder Kompressionsfeder welche sich bei der Ausschüttung entspannt und eine Kolbenhülse antreibt. Die Induktivität wird über die Resonanzfrequenz eines LC-Schwingkreises umfassend die Kompressionsfeder bestimmt. Der Autoinjektor umfasst eine elektronische Anzeige zur Signalisierung eines Injektionsendes an den Nutzer, wobei dieser Zeitpunkt um eine einstellbare Haltezeit nach der Detektion des Ausschütt-Endes liegen kann.

WO 2017/114768 offenbart einen Injektionspen mit Federantrieb mit einer auswechselbaren Karpule. Die Dosis ist durch den Benutzer manuell einstellbar. Der Injektionspen umfasst einen Rotationssensor umfassend einen gehäusefesten Teil und einen rotierbaren Teil. Eine Auswertelektronik im Pen bestimmt eine eingestellte oder ausgeschüttete Dosis basierend auf einer Relativbewegung zwischen dem ersten und zweiten Teil.

WO 2008/037801 offenbart einen Autopen mit auswechselbarer Karpule. Mit einem rotierbaren Dosiseinstellknopf kann eine gewünschte Dosis eingestellt werden. Dabei werden scheibenförmige Elemente rotiert wodurch sich eine elektrische Kapazität ändert. Eine Sensoreinheit kann anhand der Kapazitätsänderung eine Rotation der Elemente detektieren. Der Autopen umfasst weiter ein Axialsensor zum Erfassen einer axialen Position der scheibenförmigen Elemente. Anhand der Rotations- und Axialposition bestimmt eine Auswertelektronik eine eingestellte Dosis.

WO 2006/069455 A1 offenbart einen Injektionspen mit einstellbarer Dosis und Federantrieb. Der Ausschüttmechanismus umfasst ein Stirnrad, mit dem eine Drehbewegung einer Kupplungshülse an einen Encoder im Injektionspen übertragen wird. Anhand der erfassten Drehbewegung wird eine abgegebene Dosis detektiert.

WO 2014/139916 A1 offenbart einen Injektionspen mit einstellbarer Dosis und einer Karpule. Zum Einstellen einer Dosis wird ein Dosierelement zusammen mit einer Antriebshülse aus einem Pengehäuse geschraubt. Beim Ausschütten werden das Dosierelement und die Antriebshülse rotativ gekoppelt. Am Ende der Ausschüttung gibt ein Signalmechanismus dem Benutzer ein akustisches Signal, dass der Injektionspen von der Injektionsstelle entfernt werden kann.

WO 2017/114909 A1 offenbart ein Injektionsgerät mit einer Karpuleneinheit und einem Gehäuse mit einer Antriebseinheit mit einem Elektromotor. Vor der Injektion wird die Karpuleneinheit in das Gehäuse eingeführt. Das Injektionsgerät umfasst Sensoren zum detektieren einer Position einer Nadelschutzhülse der Karpuleneinheit.

WO 2019/121612 A1 beschreibt ein Injektionsgerät und ein Zusatzmodul (Add-On), welches lösbar auf einem Gehäuse des Injektionsgeräts aufsetzbar ist. Mittels Magnetfeldsensoren detektiert das Zusatzmodul eine Position einer Kolbenstange des Injektionsgeräts um den Start und das Ende einer Injektion zu erfassen.

WO 2017/089259 A1 offenbart einen Injektionspen mit einem Nadelschutz, welcher zusammen mit einer Penkappe mittels Drehbewegung vom Injektionspen entfernbar ist.

WO 2019/036576 A1 offenbart einen Injektionspen mit einem externen, separaten Zusatzmodul, welches lösbar auf den Injektionspen aufsetzbar ist und einen Rotationssensor umfasst. Mittels Sensor kann das aufgesetzte Zusasatzmodul eine eingestellte und/oder ausgeschüttete Dosis erkennen.

WO 2002/064196 A1 beschreibt einen Injektionspen mit einem Hallsensor, welcher sich im Dosierknopf befindet um eine Rotation während dem Drücken des Injektionsknopfs zu erfassen, so dass eine ausgeschüttete Dosis erfasst werden kann.

WO 2010/091522 A1 offenbart einen Injektionspen mit einer Abzughilfe zum Entfernen einer Nadelschutzkappe. Zwischen der Schutzkappe und einem Gehäuse besteht eine Sollbruchstelle, so dass beim Entfernen der Schutzkappe mit der Abzughilfe die Sollburchstelle bricht.

CN102065933 B1 offenbart einen Injektor mit einem Spritzeneinheit und einem Gehäuseteil mit dem Ausschüttmechanismus. Dabei kann die Spritzeneinheit vom Gehäusteil getrennt werden. Die Spritzeneinheit umfasst eine Spritze mit dem Medikament, welche nach Gebrauch entsorgt wird. Der Gehäusteil mit dem Ausschüttmechanismus ist für einen mehrmaligen Gebrauch vorgesehen.

Der Begriff "Produkt", "Medikament" oder "medizinische Substanz" umfasst im vorliegenden Zusammenhang jede fliessfähige medizinische Formulierung, welche geeignet ist zur kontrollierten Verabreichung mittels einer Kanüle oder Hohlnadel in subkutanes oder intramuskuläres Gewebe, beispielsweise eine Flüssigkeit, eine Lösung, ein Gel oder eine feine Suspension enthaltend einen oder mehrere medizinische Wirkstoffe. Ein Medikament kann also eine Zusammensetzung mit einem einzigen Wirkstoff oder eine vorgemischte oder coformulierte Zusammensetzung mit mehreren Wirkstoffen aus einem einzelnen Behälter sein. Der Begriff umfasst insbesondere Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form. Der Begriff umfasst weiter auch Polysaccharide, Vakzine, DNS oder RNS oder Oligonukleotide, Antikörper oder Teile von Antikörpern sowie geeignete Basis-, Hilfs- und Trägerstoffe.

Der Begriff "distal" bezeichnet eine zum vorderen, einstechseitigen Ende der Verabreichungsvorrichtung beziehungsweise zur Spitze der Injektionsnadel hin gerichtete Seite oder Richtung. Demgegenüber bezeichnet die Angabe "proximal" eine zum hinteren, dem einstechseitigen Ende gegenüberliegenden Ende der Verabreichungsvorrichtung hin gerichtete Seite oder Richtung.

Unter den Begriffen "Injektionssystem" oder "Injektor" wird in der vorliegenden Beschreibung eine Vorrichtung verstanden, bei der die Injektionsnadel nach erfolgter Abgabe einer kontrollierten Menge der medizinischen Substanz aus dem Gewebe entfernt wird. Somit verbleibt bei einem Injektionssystem oder bei einem Injektor im Unterschied zu einem Infusionssystem die Injektionsnadel nicht über einen längeren Zeitraum von mehreren Stunden im Gewebe.

### DARSTELLUNG DER ERFINDUNG

Es ist Aufgabe der Erfindung einen Autoinjektor mit Torsionsfederantrieb zum automatischen Ausschütten einer Flüssigkeit aus einem Produktbehälter anzugeben, welcher es ermöglicht, eine axiale Position eines Kolbens im Behältnis genau und/oder energiesparend zu ermitteln. Die Aufgabe wird gelöst durch einen Autoinjektor mit den Merkmalen des unabhängigen Anspruchs 1. Bevorzugte Ausführungsformen der Erfindung sind Gegenstand der abhängigen Ansprüche.

Ein erfindungsgemässer Autoinjektor umfasst ein Gehäuse mit einer Längsachse, einen Produktbehälter, eine zur einmaligen Ausschüttung eines maximalen Inhalts des Produktbehälters vorgespannte Torsionsfeder, ein Antriebselement, und ein Vortriebselement. Zur Ausschüttung einer maximalen oder zumindest einer vorbestimmten Flüssigkeitsmenge aus dem Produktbehälters versetzt die Torsionsfeder das Antriebselement in Rotation um die Längsachse, und das rotierende Antriebselement bewirkt eine Vortriebsbewegung des Vortriebselements zur Verschiebung eines Kolbens im Produktbehälter. Der Autoinjektor umfasst weiter einen permanent eingebauten Rotationssensor zur abwechslungsweisen und kontinuierlichen Detektion von mindestens zwei Rotationspositionen pro Umdrehung des Antriebselementes während der Ausschüttung, sowie eine Prozessoreinheit zur Bestimmung einer axialen Kolbenposition des Kolbens im Produktbehälter oder eines Restvolumens im Produktbehälter aus den sukzessive detektierten Rotationspositionen. Erfindungsgemäss wird eine während der Ausschüttung durch die Torsionsfeder unmittelbar bewirkte Rotation eines Antriebsglieds kontinuierlich und mit einer Auflösung von einer halben Umdrehung oder weniger gemessen und daraus der Vortrieb und die Kolbenposition bestimmt. Durch die verbesserte Auflösung kann das Restvolumen beziehungsweise die ausgeschüttete Menge an Medikament genau bestimmt werden, was insbesondere in Fällen von Bedeutung ist, in welchen die Ausschüttung nicht wie geplant verläuft oder gar durch den Anwender abgebrochen wird. Der Fortschritt einer Ausschüttung kann zudem in Echtzeit erfasst werden, und insbesondere Schwankungen in der Vortriebsgeschwindigkeit sind ohne Verzögerung erkennbar; ebenso sind aussagekräftige Rückschlüsse auf eine Stopfensilikonisierung im Rahmen von Qualitätsüberwachungen bei gealterten Autoinjektoren ableitbar. Diese Erkenntnisse wie auch weitere Injektionsdaten können anschliessend einer zentralen Auswertung zugeführt werden und im Rahmen einer umfassenden Überwachung einer Flotte von Autoinjektoren untereinander und mit Referenzwerten verglichen werden.

In einer bevorzugten Ausführungsform weist das Vortriebselement einen nichtrotationssymmetrischen Querschnitt auf mit einem Axialführungselement in Form einer Nut, Ebene, oder Rippe parallel zur Längsachse, über welche das Vortriebselement durch das Gehäuse oder ein im Gehäuse rotationsfest aufgenommenes Führungselement axial linear geführt ist. Das Vortriebselement ist dabei über ein Gewinde an das Antriebselement gekoppelt und bewegt sich ausschliesslich axial in Ausschüttrichtung und nicht etwa in einer schraubenden Vortriebsbewegung. Dadurch wird Reibung zwischen einer distalen Stirnfläche des Vortriebsglieds und dem Kolben vermieden, zudem ist es einfacher, das Antriebselement und/oder das Vortriebselement mit einem geänderten Gewinde oder mit einer speziellen Gewindeflächenbeschichtung zu versehen als das Gehäuse. Auch kann eine nur teilweise initiale Verschraubung von Antriebselement und Vortriebselement genutzt werden, um die Ausgangsposition des Vortriebselements an unterschiedlichste Füllvolumina anzupassen. Dazu sind keine Varianten des Vortriebselements notwendig, insbesondere falls die Ausschüttung durch eine Freigabe der Rotation des axial festen Antriebselements gestartet wird.

Vorteilhafterweise weist das Antriebselement eine Gewindestange mit einem Aussengewinde und das Vortriebselement eine Hülse mit dem Axialführungselement und einem an das Aussengewinde angepassten Innengewinde auf. Das Innengewinde erstreckt sich über eine dem Ausschütthub entsprechende Länge, und das Aussengewinde ist am proximalen Ende der Gewindestange auf wenige Windungen verkürzt oder umfasst ein Gewindesegment von weniger als einer Windung. Alternativ dazu erstreckt sich das Aussengewinde über die Länge des Ausschütthubs und ist das Innengewinde entsprechend verkürzt. Bei Rotation der Gewindestange wird die nichtrotierend gelagerte Hülse in distale Richtung geschoben, was einfacher zu bewerkstelligen ist als die Linearführung einer Gewindestange.

Die Torsionsfeder als elastisches Mittel zur Erzeugung eines Drehmoments ist bevorzugt eine Spiralfeder, kann aber auch als Blattfeder, Triebfeder, Konusfeder, Wendeltorsionsfeder, Drehstab oder Kombinationen davon ausgebildet sein. Sie ist bei Lieferung beziehungsweise vor Inbetriebnahme des Autoinjektors maximal oder hinreichend vorgespannt für eine einmalige Ausschüttung des gesamten oder zumindest eines vorbestimmten Inhalts des Produktbehälters. Der Autoinjektor verfügt dementsprechend nicht über einen Dosiswahlmechanismus. Eine vorgefüllte Einweg-Fertigspritze umfasst den Produktbehälter und eine daran unlösbar befestigte Injektionsnadel und ist axial fixiert im Gehäuse des Autoinjektors gehalten. Der Autoinjektor, oder zumindest die Fertigspritze und der Spritzenhalter, sind entsprechend nur für einen einmaligen Gebrauch vorgesehen.

In einer bevorzugten Ausführungsform umfasst der Autoinjektor eine optische, akustische, oder taktile Signalisierungs- oder Anzeigeeinheit, welche von der Prozessoreinheit elektronisch angesteuert oder aktiviert wird. Dies geschieht nach Bestimmen einer axialen Kolbenposition oder zumindest einer kumulierten Drehbewegung des Antriebselements welche einer zumindest annähernd vollständigen Ausschüttung entspricht, und/oder nach einem als Ende der Ausschüttung registrierten Ausbleiben eines Rotationssensorsignals, sowie nach anschliessendem Ablauf einer vorbestimmten Haltezeit. Die Signalisierungseinheit erzeugt ein Signal welches ein Ende der Injektion anzeigt und dem Anwender bestätigt, dass der Autoinjektor jetzt sicher von der Einstichstelle wegbewegt werden kann. Dieses Signal wird spätestens bei Entfernen des Autoinjektors von der Einstichstelle quittiert und beendet. Die Haltezeit beträgt typischerweise einige wenige Sekunden, beispielsweise zwischen 2 und 15 Sekunden und bevorzugt zwischen 3 und 10 Sekunden, und stellt sicher, dass die injizierte Menge an Medikament vollständig vom subkutanen Gewebe aufgenommen wird und keine Flüssigkeit nach Wegbewegen des Autoinjektors durch die Einstichstelle auf die Gewebeoberfläche gelangt. Dementsprechend verfügt der Autoinjektor nicht über einen rein mechanisch ausgelösten Endklick, bei welchem ein Anschlagelement durch eine Feder beschleunigt wird, insbesondere wird die Ausschüttfeder nach Bestimmen des Ausschüttendes nicht mehr weiter entspannt, sie ist dazu spätestens nach Beginn der Ausschüttung in Entspannungsrichtung gehäusefest abgestützt. Dadurch wird vermieden, dass eine zwischen der Ausschüttfeder und dem Gehäuse angeordnete Komponente wie beispielsweise das Anschlagelement während langer Zeit, und insbesondere während einer Lagerdauer, mit einer maximalen Federkraft belastet wird.

In einer bevorzugten Ausführungsform umfasst der Autoinjektor eine Nadelschutzhülse, welche in einer Sicherungsbewegung beim Entfernen des Autoinjektors von der Einstichstelle aus einer proximalen Endposition durch eine Nadelschutzfeder in eine distale Endposition zur Abdeckung einer Injektionsnadel bewegt wird. Ein Nadelschutzhülsendetektor ist ausgebildet zur Detektion dieser Sicherungsbewegung oder zumindest der Tatsache, dass die Nadelschutzhülse sich nicht mehr in der proximalen Endposition befindet. Zum Zeitpunkt der Detektion der Sicherungsbewegung bestimmt die Prozessoreinheit eine Kolbenposition oder eine zugehörige Flüssigkeitsmenge, entweder eine Restmenge an Flüssigkeit im Produktbehälter oder eine bis zu diesem Zeitpunkt ausgeschüttete Menge. Zumindest bei einer festgestellten Restmenge oberhalb einer maximalen Restmenge wird Erstere von der Prozessoreinheit abgespeichert und/oder einem Nutzer angezeigt. Dadurch besteht die Möglichkeit nach einem Abbruch der Injektion durch vorzeitiges Entfernen des Autoinjektors von der Einstichstelle noch festzustellen, welche Mengen an Medikament tatsächlich in das Gewebe ausgeschüttet wurden, und es können entsprechende Massnahmen getroffen werden.

In einer bevorzugten Ausführungsform umfasst der Rotationssensor einen mit dem Antriebselement rotierenden rotationsunsymmetrisch ausgebildetem Aktor in Form eines Zahnrads oder einer Blende mit über einen Umfang verteilten Zähnen und/oder Ausnehmungen, und ein Abtastelement zur Abtastung oder Detektion der rotierenden Zähne und/oder Ausnehmungen. Über den Umfang gleichmässig verteilt sind mindestens zwei Zähne oder Ausnehmungen vorgesehen, wobei alternativ oder zusätzlich auch mehrere über den Umfang versetzt angeordnete Abtastelemente denkbar sind. Der Rotationssensor umfasst in einer ersten Variante ein durch die Zähne und/oder Ausnehmungen radial oder axial mechanisch auslenkbares Abtastelement. Bevorzugt umfasst das Abtastelement einen elektromechanischen Schalter oder einen Dehnmessstreifen, wobei ein Hebel des Schalters oder ein flexibler Träger des Dehnmessstreifens axial oder radial ausgerichtet sind. In einer zweiten Variante umfasst der Rotationssensor einen berührungsfrei agierenden Photosensor mit einer Lichtquelle und einem Photodetektor als Abtastelement zur Detektion von parallel zur Längsachse durch die Ausnehmungen der Blende propagierenden Lichts der Lichtquelle.

In einer bevorzugten Ausführungsform umfasst der Rotationssensor eine mit dem Antriebselement rotierende, rotationsunsymmetrisch ausgebildete und dazu in Sektoren unterteilte Oberfläche, wobei ein optisches Reflexionsverhalten zwischen den Sektoren ändert. Der Rotationssensor umfasst einen berührungsfrei operierenden Photosensor mit einer Lichtquelle und einem Photodetektor als Abtastelement zur Detektion von Licht der Lichtquelle, welches von einem Sektor der Oberfläche in Richtung des Photodetektors reflektiert wird. Die mindestens zwei reflektierenden Sektoren können eine helle Farbe aufweisen oder spiegelnd ausgebildet sein. Eine exemplarische Unterteilung der Oberfläche in 8 Sektoren bedeutet eine entsprechende Auflösung der Rotationsposition von 45°. Die Oberfläche kann zu einem Flansch der Federspule gehören oder zu einer Scheibe oder einem sonstigen Körper mit einer Rotationsachse parallel zur Längsachse.

In einer bevorzugten Ausführungsform umfasst der Rotationssensor einen berührungslosen elektromagnetischen Sensor und eine mit dem Antriebselement rotierende diskrete Anordnung von passiven elektromagnetischen Aktoren. Dazu gehören beispielsweise ein Hall-Sensor zusammen mit einer rotierbaren Anordnung von Permanentmagneten, oder ein induktiver Sensor zusammen mit einer rotierbaren Anordnung von induktiv detektierbaren elektrischen Leitern.

Der Autoinjektor umfasst eine Kommunikationseinheit zur Kommunikation mit einem mobilen oder stationären Drittgerät und/oder eine Anzeigeeinheit zur Anzeige eines Zustandes des Autoinjektors. Die Prozessoreinheit, die Kommunikationseinheit und/oder die Anzeigeeinheit, und eine Energiequelle zu deren Speisung sind auf einer Leiterplatine angeordnet, welche zusammen mit einem bevorzugt durch vorgegebene Sollbruchlinien im Gehäuse einfach abtrennbaren Gehäuseteil vom Rest des Autoinjektors entfernt werden kann. Bevorzugt sind auch alle entsorgungskritischen elektronischen Komponenten des Rotationssensors auf der Leiterplatine angeordnet, so dass keine elektronischen Bauteile nach Entfernen der Leiterplatine im Autoinjektor zurückbleiben.

In einer bevorzugten Ausführungsform umfasst der Autoinjektor eine Gerätekappe, eine Nadelschutzhülse und eine Nadelschutzfeder, wobei in einem Liefer- oder Ausgangszustand die Nadelschutzhülse durch die Nadelschutzfeder in einen ersten Anschlag gedrängt wird. Nach Abziehen der Gerätekappe aber vor der Ausschüttung, in einem zur Injektion bereiten, entsicherten Zustand, wird die Nadelschutzhülse durch die Nadelschutzfeder in einen zweiten Anschlag mit dem Gehäuse, dem Spritzenhalter, oder einem axialfesten Mechanikhalter gedrängt, so dass die Nadelschutzhülse sich in einer vorderen Endposition weiter distal befindet als im Lieferzustand. Der Autoinjektor umfasst weiter einen Axialpositionsdetektor zur Detektion dieser initialen Bewegung der Nadelschutzhülse in distale Richtung in den zweiten Anschlag oder zur Detektion der Nadelschutzhülse im zweiten Anschlag. Dadurch kann ein Gerätekappenabzug indirekt, das heisst nicht unmittelbar an der Gerätekappe selbst und insbesondere durch einen in der proximalen oder hinteren Hälfte des Autoinjektors angeordneten Detektor detektiert werden. Die distale Hälfte des Autoinjektors, und insbesondere der Bereich der Fertigspritze, bleibt dadurch frei von elektronischen Bauteilen und/oder elektrischen Leitern. Alternativ dazu könnte die An-/Abwesenheit einer überlangen, bis in die proximale Hälfte des Autoinjektors reichenden Gerätekappe unmittelbar mit einem in oder an der proximalen Gerätehälfte angeordneten Kappendetektor erkannt werden.

Der erfolgte Gerätekappenabzug kann dem Anwender geeignet signalisiert werden, und/oder eine Sensoreinheit, wie beispielsweise der besagte Rotationssensor oder ein anderer Vortriebssensor im Falle eines linearen Antriebs durch eine Druckfeder, wird durch den detektierten Gerätekappenabzug aktiviert, so dass er für einen unmittelbar bevorstehenden Einsatz bereit ist und nicht permanent aktiviert sein muss oder erst durch die zu detektierende Bewegung selbst aktiviert werden muss. Handelt es sich bei dem Axialpositionsdetektor um einen elektromechanischen Schalter kann durch Bewegen eines Kontaktelements oder Kippen eines Schalthebels ein Kontakt geschlossen werden, was durch ein Prozessorelement im Energiesparmodus erkannt und zur Aktivierung von zuvor stromlosen Komponenten oder Bauteilen genutzt werden kann. Elektronische Komponenten eines Elektronikmoduls des Autoinjektors, welche wie vorliegend beschrieben aktiviert werden, sind also über ihren Lebenszyklus umfassend die Lagerdauer und den Einsatz während einer Injektion insgesamt energiesparend betrieben. Eine Batterie oder sonstige Energiespeichereinheit zu ihrer Versorgung kann entsprechend knapp dimensioniert werden. Nach erfolgter Detektion des Gerätekappenabzugs kann durch das Elektronikmodul ein umfassender Selbsttest durchgeführt werden, umfassend beispielsweise eine Prüfung von Datum, Therapieplan, Temperaturhistorie, aktueller Temperatur, Nutzerinformation, und/oder Status einer Kommunikationsverbindung.

In einer bevorzugten Ausführungsform wird die Nadelschutzhülse bei Kontakt und Anpressen an eine Injektionsstelle in proximale Richtung gegenüber dem Gehäuse, und unter Spannung der Nadelschutzfeder in eine hintere Endposition bewegt und dadurch eine Ausschüttung gestartet oder ermöglicht. Wahlweise kann ein bei dieser Gelegenheit erzeugtes Signal des Axialpositionsdetektors oder ein erstes Signal des Rotationssensors als Start der Ausschüttung definiert und registriert werden. Am Ende der Injektion, wenn der Autoinjektor wieder von der Injektionsstelle wegbewegt wird, bewegt sich die Nadelschutzhülse wieder in die distale Richtung in eine Nadelschutzposition, welche mit der ersten Endposition zusammenfallen kann. Bevorzugt detektiert der Axialpositionsdetektor auch diese finale Bewegung der Nadelschutzhülse in die Nadelschutzposition, woraus die Prozessoreinheit feststellen kann, ob die Haltezeit nach dem Ende der Ausschüttung korrekt eingehalten wurde.

Ein Autoinjektor nach der Erfindung umfasst ein Gehäuse, eine im Gehäuse axial fest angeordnete Fertigspritze oder sonstigen Produktbehälter, eine bei Lieferung des Autoinjektors vorgeladene Ausschüttfeder in Gestalt einer Druck- oder Torsionsfeder zur einmaligen und vollständigen Ausschüttung einer Flüssigkeitsmenge aus dem Produktbehälter, ein durch die Ausschüttfeder angetriebenes Vortriebselement zum Vortrieb eines Kolbens im Produktbehälter, eine Sensoreinheit zur Bestimmung einer axialen Kolbenposition des Kolbens im Produktbehälter, und eine Signalisierungseinheit, welche von einer Prozessoreinheit nach Bestimmen einer axialen Kolbenposition entsprechend einer zumindest annähernd vollständigen Ausschüttung und nach anschliessendem Ablauf einer vorbestimmten Haltezeit zur Signalisierung eines Endes der Injektion angesteuert wird. Ein Ende der Ausschüttfeder stützt sich permanent am Gehäuse ab, die Ausschüttfeder entspannt sich dementsprechend ausschliesslich zum Vortrieb des Kolbens. Ein proximales Ende einer Wendel- oder Druckfeder, oder ein äusseres Ende einer Spiralfeder ist mit der proximalen Endkappe des Gehäuses fest verbunden. Dadurch wird vermieden, dass eine zwischen der Ausschüttfeder und dem Gehäuse angeordnete Komponente wie beispielsweise ein mechanisches Anschlagelement während langer Zeit, und insbesondere während einer Lagerdauer, mit einer maximalen Federkraft belastet wird. Das Ende der Ausschüttung oder der Beginn der Haltezeit wird höchstens über die elektronische Signalisierungseinheit angezeigt, oder kann auch ganz unterbleiben. Dadurch wird ein Nutzer nicht durch ein weiteres Signal vor dem verzögerten Signal verwirrt, welches ihm das Ende der Injektion inklusive Haltezeit anzeigt und das Entfernen des Autoinjektors von der Injektionsstelle erlaubt.

Dementsprechend ist ein Autoinjektor mit einem Gehäuse, einem Produktbehälter, einer Ausschüttfeder zur einmaligen und insbesondere vollständigen Ausschüttung einer Flüssigkeitsmenge aus dem Produktbehälter, einem durch die Ausschüttfeder angetriebenen Vortriebselement, einer Sensoreinheit zur Bestimmung einer axialen Kolbenposition eines Kolbens im Produktbehälter, und einer Signalisierungseinheit, welche von einer Prozessoreinheit nach Bestimmen einer axialen Kolbenposition entsprechend einer zumindest annähernd vollständigen Ausschüttung und nach anschliessendem Ablauf einer vorbestimmten Haltezeit zur Signalisierung eines Endes der Injektion angesteuert wird, gekennzeichnet dadurch, dass ein Ende der Ausschüttfeder sich permanent am Gehäuse abstützt.

### FIGUREN

In Zusammenhang mit den angehängten Figuren werden nachfolgend bevorzugte Ausführungsformen der Erfindung beschrieben. Diese sollen grundsätzliche Möglichkeiten der Erfindung aufzeigen und keinesfalls einschränkend ausgelegt werden. Es zeigen
- Fig.1: einen Autoinjektor mit Elektronikmodul;
- Fig.2: einen ersten Rotationssensor mit Gabel-Lichtschranke;
- Fig.3: einen zweiten Rotationssensor mit Reflex-Lichtschranke;
- Fig.4: einen dritten Rotationssensor mit elektromechanischem Schalter;
- Fig.5: einen vierten Rotationssensor mit Hall-Sensor;
- Fig.6: einen Autoinjektor mit Elektronikmodul und Axialpositionsdetektor;
- Fig.7: einen ersten Axialpositionsdetektor mit elektromechanischem Schalter;
- Fig.8: einen zweiten Axialpositionsdetektor mit Gabel-Lichtschranke;
- Fig.9: einen dritten Axialpositionsdetektors mit Reflexlichtschranke;
- Fig. 10: einen vierten Axialpositionsdetektors mit Reflexlichtschranke;
- Fig. 11: einen fünften Axialpositionsdetektor mit Schleifkontaktsensor; und
- Fig. 12: zwei Autoinjektoren mit Sollbruchlinie.

### FIGURENBESCHREIBUNG

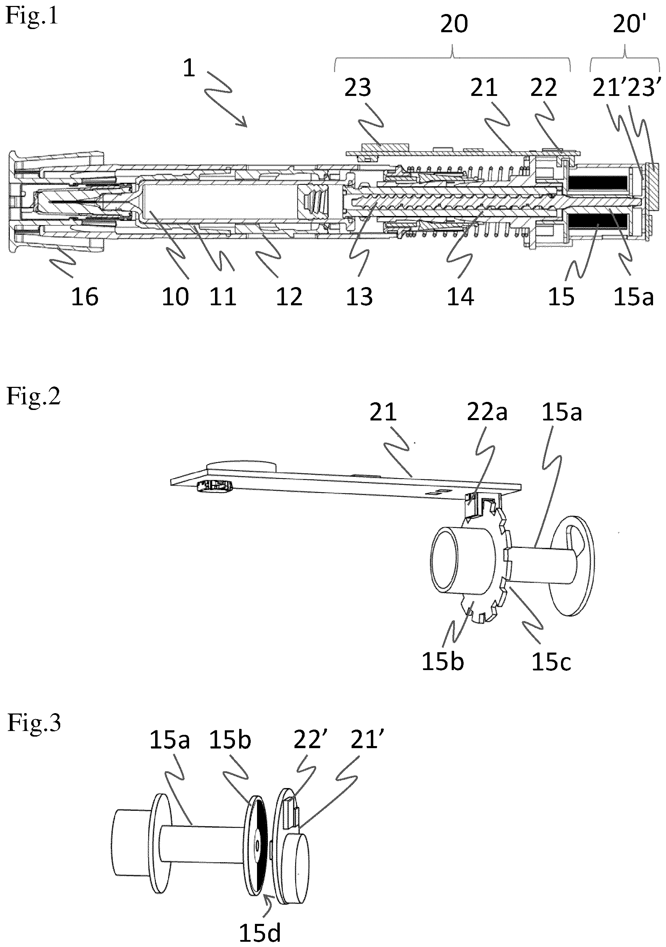

Fig.1 zeigt einen partiellen Längsschnitt durch einen Autoinjektor 1 entlang seiner Längsachse, umfassend eine Fertigspritze 10, einen Spritzenhalter 11, eine Nadelschutzhülse 12, ein Antriebselement 13 in Form einer Gewindestange, ein Vortriebselement 14 in Form einer Hülse mit einem Innengewinde in Eingriff mit dem Gewinde der Gewindestange, und einer Torsionsfeder in Gestalt einer Trieb- oder Spiralfeder 15, welche auf einer Federspule 15a aufgewickelt ist. Die Federspule 15a ist rotationsfest und bevorzugt axialfest mit dem Antriebselement 13 gekoppelt, Federspule und Antriebselement können somit auch einstückig ausgebildet sein. Ein die genannten Bestandteile allseitig umgebendes und zumindest den Spritzenhalter und die Triebfeder axialfest aufnehmendes Gerätegehäuse ist nicht dargestellt, dafür eine Gerätekappe 16 welche vor der Verabreichung zusammen mit einer Nadelschutzhülse vom Autoinjektor abgezogen wird. Ferner ist schematisch gezeigt ein Elektronikmodul 20 mit einer seitlichen Leiterplatine 21 welche seitlich neben dem Antriebselement angeordnet ist, sowie ein alternatives Elektronikmodul 20' mit einer proximalen Leiterplatine 21', welche proximal der Torsionsfeder 15 angeordnet ist. Auf den Leiterplatinen sind alternative Rotationssensoren 22 montiert wie weiter unten beschrieben, sowie eine Prozessoreinheit und eine Kommunikationseinheit samt einer Energiequelle 23, 23' zu ihrer Speisung. Die Leiterplatinen sind in dem nicht dargestellten Gerätegehäuse des Autoinjektors fest angeordnet.

Fig.2 zeigt die Federspule 15a, einen proximalen Abschnitt der seitlichen Leiterplatine 21, und einen Rotationssensor umfassend eine auf der Leiterplatine angeordnete Gabel-Lichtschranke 22a in perspektivischer Ansicht. Die Federspule umfasst einen distalen Flansch 15b in Form einer konzentrisch und senkrecht zur Längsachse angeordneten Blende mit einer radialen Modulation 15c des Flanschumfangs in Form von über den Umfang verteilt angeordneten Ausnehmungen oder Einbuchtungen. Die Gabel-Lichtschranke ist so gestaltet, dass Licht von einer integrierten Lichtquelle auf einer Innenseite der Gabel über eine Lichtstrecke auf einen integrierten Lichtdetektor auf einer gegenüberliegenden Innenseite gelangen kann. Die Lichtschranke ist mit der Lichtstrecke in Längsrichtung des Autoinjektors so platziert dass bei entsprechender Ausrichtung die Lichtstrecke eine der Ausnehmungen traversiert beziehungsweise durch ein peripheres, zwei benachbarte Ausnehmungen in Umfangrichtung trennendes Segment der Blende unterbrochen wird. Eine Drehung der Blende und damit der Federspule und des Antriebselements wird detektiert durch eine stetige Abfolge von auf dem Lichtdetektor auftreffenden Lichtpulsen, welche einfach gezählt und in eine kumulierte Drehbewegung konvertiert werden können.

Alternativ zum distalen Flansch könnte auch ein proximaler Flansch der Federspule zur Rotationsdetektion beigezogen werden, oder sonst eine drehfest mit dem Antriebselement verbunden Scheibe. An Stelle der gezeigten Ausnehmungen an der Peripherie der Blende können auch auf einem achsenkonzentrischen Kreis angeordnete Schlitze oder Löcher in der Blende drehwinkelabhängig das Licht entlang der Lichtstrecke passieren lassen oder den Lichtdetektor abdecken. Falls die Lichtstrecke der Lichtschranke radial ausgerichtet ist kann die Lichtstrecke unterbrochen werden durch einen zinnen- oder kronenförmigen Abschluss einer Zylinderhülse mit einer Achse parallel zur Längsachse. Die Anzahl der Ausnehmungen, Schlitze oder Zinnen bestimmen die Auflösung der Rotationsdetektion. Eine Konfiguration mit nur einer Ausnehmung, einem Schlitz oder einer Zinne generiert bereits zwei Übergänge oder Wechsel im Signal des Detektors, und unterscheidet somit zwei Rotationspositionen pro Umdrehung des Antriebselements, was in einer gegenüber einer Detektion von ganzen Umdrehungen verbesserten Auflösung resultiert. Die Lichtquelle kann sichtbares Licht aussenden oder eine Infrarot-LED umfassen, der Lichtdetektor kann ein Foto-Transistor oder Foto-Mikrosensor sein.

Fig.3 zeigt ein proximales Ende der Federspule 15a mit einem proximalen Flansch 15b, die proximale Leiterplatine 21', und einen auf der Platine angeordneten Rotationssensor 22' umfassend eine Reflex-Lichtschranke in perspektivischer Ansicht. Auf der nach proximal gerichteten Seite des Flanschs ist ein Muster 15d mit einem lichtabsorbierenden dunklen und einem lichtreflektierenden hellen Sektor aufgetragen. Der Rotationssensor umfasst eine Lichtquelle und einen Lichtdetektor zur Detektion des von der Lichtquelle emittierten und vom hellen Sektor reflektierten Licht. Die dargestellte Konfiguration benötigt radial keinen zusätzlichen Platz.

An Stelle von weissem Licht kann die Lichtquelle auch Licht einer bestimmten Wellenlänge beziehungsweise eines Wellenlängenbereichs, inklusive IR, aussenden und der reflektierende Sektor mit Farbe derselben Wellenlänge eingefärbt sein. Alternativ oder zusätzlich zu einer unterschiedlichen Einfärbung der Sektoren kann diese Seite des Flansches auch eine abwechselnde Oberflächenbeschaffenheit aufweisen (rau und glatt) oder es können unterschiedlich geneigte Spiegelflächen aufgebracht werden, welche das Licht jeweils zum Detektor oder in eine andere Richtung lenken. Die Flächenabschnitte mit unterschiedlichen Reflexionseigenschaften können auch auf einer Innen- oder Aussenfläche einer Zylinderhülse mit einer Achse parallel zur Längsachse angebracht sein, wobei die Reflex-Lichtschranke radial von innen oder aussen auf das drehende Muster ausgerichtet ist.

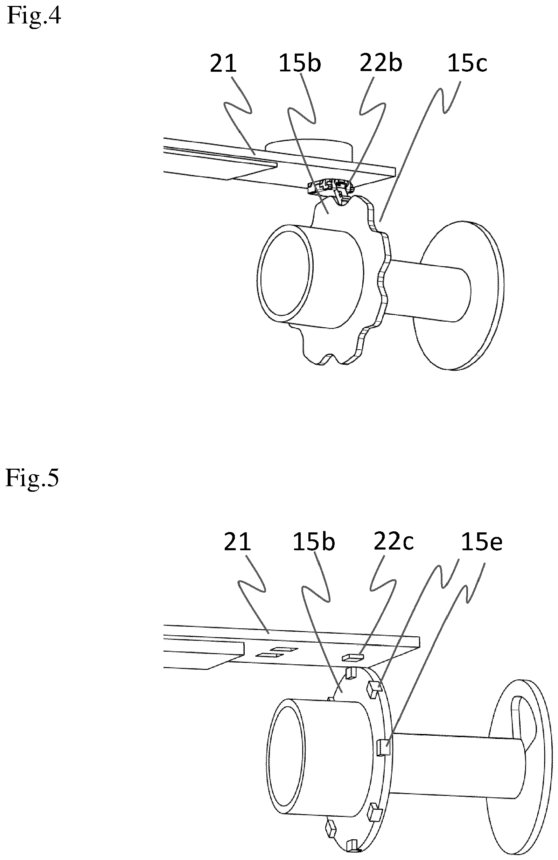

Fig.4 zeigt die Federspule, einen proximalen Abschnitt der seitlichen Leiterplatine 21, und einen Rotationssensor umfassend einen elektromechanischen Schalter 22b in perspektivischer Ansicht. Wiederum umfasst der distale Flansch 15b der Federspule eine radiale Modulation 15c des Flanschumfangs in Form von Wellen oder Zähnen, welche einen Hebel des Schalters bei einer Drehbewegung radial verkippen. Der Hebel kehrt nach einer Auslösung selbsttätig in seine unbetätigte Position zurück und ist dadurch bereit für eine nächste Auslösung. Alternativ zur Modulation des Flanschumfangs können auf einer Stirnfläche des distalen Flansches 15b der Federspule radial ausgerichtete Rippen oder Vertiefungen angebracht sein, welche ihrerseits einen axial auslenkbaren Hebel eines Schalters bei einer Drehbewegung verkippen.

An Stelle des elektromechanischen Schalters kann auch ein entsprechend positionierter Dehnmessstreifen zur Erfassung einer Rotationsbewegung der Strukturen des jeweiligen Flansches eingesetzt werden. Jede einzelne Rippe beziehungsweise jede Einbuchtung des Flansches drückt dabei ein Ende des Dehnmessstreifens tangential zur Seite oder radial nach aussen, so dass die dadurch entstehende mechanische Spannung im Dehnmesstreifen detektiert werden kann. Der Dehnmesstreifen selbst ist bevorzugt auf einem Arm am proximalen Ende der Leiterplatine angeordnet, welcher selbst axial oder tangential ausgerichtet ist und dadurch radial beziehungsweise senkrecht zur Ebene der Platine ausgelenkt werden kann. Der Arm ist durch Ausnehmungen oder Schlitze im Trägermaterial der Leiterplatine geformt, wodurch die Flexibilität des Arms kontrolliert werden kann. An Stelle des Dehnmessstreifens ist auch ein Piezo-Element denkbar, welches durch die Auslenkung des Arms geeignet gestaucht oder gedehnt wird.

Fig.5 zeigt die Federspule, einen proximalen Abschnitt der seitlichen Leiterplatine 21, und einen Rotationssensor umfassend einen Hall-Sensor 22c in perspektivischer Ansicht. Auf dem distalen Flansch 15b der Federspule sind dazu über den Umfang verteilt acht Permanentmagnete 15e angeordnet, deren Anwesenheit beziehungsweise deren Passage von dem Hall Sensor 22c auf der Platine erkannt wird. An Stelle der diskret angeordneten Magnete kann auch ein permanentmagnetischer Ring mit unterschiedlich orientierten magnetischen Segmenten vorgesehen sein. Ebenfalls denkbar sind diskret angeordnete magnetisierbare Elemente oder ein Ring aus magnetisch aktivem Material mit einer über den Umfang modulierten Dicke jeweils zusammen mit einem neben dem Hall Sensor fix platzierten Permanentmagneten, dessen magnetisches Feld am Ort des Sensors durch die An-/Abwesenheit des magnetisierbaren Materials beeinflusst wird. An Stelle des Hall-Sensors kann auch ein Reed-Schalter oder ein magneto-resistiver Widerstand verwendet werden. Ebenfalls denkbar ist ein Rotationssensor auf Basis von Schleifkontakten und rotierenden Leiterabschnitten.

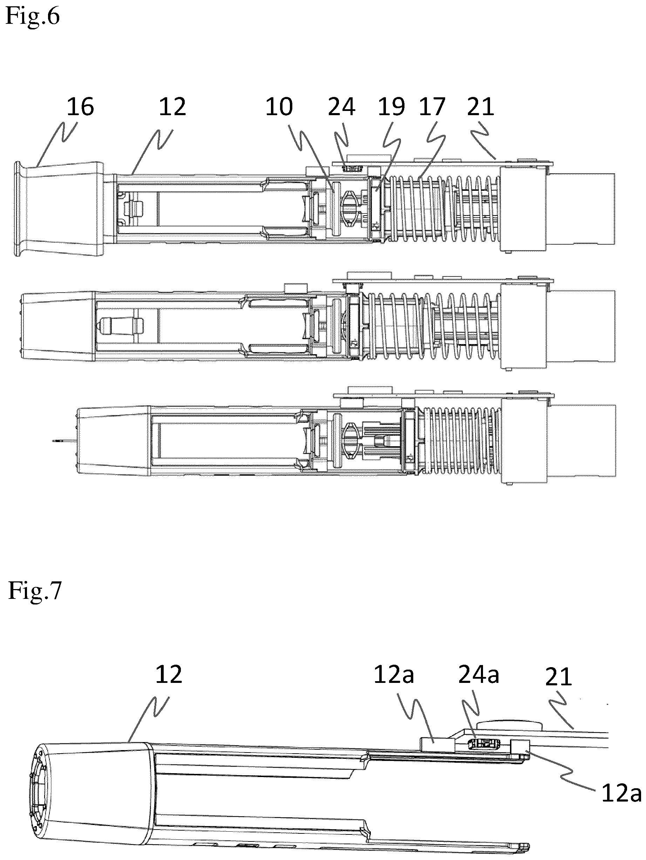

Fig.6 zeigt eine Ansicht eines Autoinjektors gemäss einem zweiten Aspekt der Erfindung, wobei das Gerätegehäuse wiederum weggelassen wurde. Erkennbar sind die Gerätekappe 16, die Nadelschutzhülse 12, ein Fingerflansch der Fertigspritze 10, die Nadelschutzfeder 17, und eine seitliche Leiterplatine 21 mit darauf montiertem Axialpositionsdetektor 24 für die Nadelschutzhülse. In Fig.6 oben befinden sich der Autoinjektor in einem Lieferzustand und die Nadelschutzhülse 12 in einer Ausgangsposition. Die Gerätekappe 16 ist mittels geeigneter Nocken oder lösbarer Schnapper am Gehäuse des Autoinjektors axial fest verankert. Die Nadelschutzfeder 17 drückt eine erste nach distal gerichtete starre Fläche oder Kante der Nadelschutzhülse in einen ersten Anschlag, welcher unmittelbar durch die Gerätekappe gebildet ist. Der erste Anschlag kann auch am Gehäuse, am Spritzenhalter, oder an einem Mechanikhalter des Autoinjektors vorgesehen sein und eine schräge, nicht senkrecht zur Längsachse ausgerichtete Anschlagfläche aufweisen. In diesem Fall ist ein radial flexibles Sperrelement der Nadelschutzhülse in Eingriff mit dem ersten Anschlag, und wird durch die montierte Gerätekappe in diesem Eingriff gesichert. Dadurch wird verhindert, dass die Kraft der Nadelschutzfeder im Auslieferzustand auf die Gerätekappe wirkt und zu einer ungewollten Bewegung der Nadelschutzkappe und dadurch zu einer Beeinträchtigung der Sterilität der Nadel führt. Nach Abzug der Gerätekappe wird das Sperrelement über die schräge Anschlagfläche aus dem Eingriff gedrängt. Falls die Nadelschutzhülse 12 kraftschlüssig mit einer Schalthülse 19 verschnappt oder gar einstückig mit dieser ausgebildet ist kann an Stelle der Nadelschutzhülse auch die Schalthülse 19 wie beschrieben über das distale Ende der Nadelschutzfeder 17 in den ersten Anschlag gedrängt werden.

Nach einem Abzug der Gerätekappe 16 unter Lösung der Nocken oder Schnapper bewegt sich die Nadelschutzhülse 12 durch die expandierende Nadelschutzfeder 17 nach distal. In Fig.6 Mitte befindet sich der Autoinjektor in einem entsicherten Zustand bereit zum Einstechen und Ausschütten. Die Nadelschutzfeder drückt eine zweite nach distal gerichtete Fläche der Nadelschutzhülse oder der Schalthülse gegen einen zweiten Anschlag des Gehäuses, die Nadelschutzhülse befindet sich in einer distalen Endposition. Der zweite Anschlag kann auch durch den Spritzenhalter, den Mechanikhalter, oder sonst eine axialfest mit dem Gehäuse angebrachte Komponente gebildet werden.

Zur Injektion wird das distale Ende der Nadelschutzhülse gegen die Einstichstelle gedrückt, dadurch wird die Nadelschutzhülse unter Kompression der Nadelschutzfeder in proximale Richtung in das und gegenüber dem Gehäuse verschoben. Zur Einstellung einer Einstechtiefe in einem Bereich von 5 bis 8 mm, und insbesondere einer Verkürzung, kann dabei der proximale Anschlag der Nadelschutzhülse am Gehäuse oder der proximale Anschlag der Schalthülse am Mechanikhalter durch geeignet angebrachte kurze axiale Rippen oder Vorsprünge an einer der beiden anschlagenden Komponenten definiert werden. Gleichzeitig sind die an der Schalthülse angebrachten oder mit der Schalthülse zusammenwirkenden Auslöseelemente für die Auslösung der Ausschüttung axial angepasst zu positionieren. In Fig.6 unten befindet sich der Autoinjektor in einem eingestochenen Zustand, mit der Nadelschutzhülse in einer hinteren Endposition. Nach erfolgter Ausschüttung wird der Autoinjektor wieder von der Einstichstelle abgehoben, wodurch sich die Nadelschutzhülse wieder nach distal bewegt bis in die vordere Endposition, in welcher sie durch einen Sicherungsmechanismus gegen erneutes Einschieben gesichert wird.

Der Axialpositionsdetektor 24 detektiert die Nadelschutzhülse 12 in der vorderen und in der hinteren Endposition. Alternativ zur Detektion der axialen Endpositionen kann auch eine axiale Bewegung oder der Übergang der Nadelschutzhülse von Ausgangs- in die vordere Endposition oder von der vorderen in die hintere Endposition registriert werden. Falls sich die Spritze durch einen Einstechmechanismus selbst gegenüber dem Gehäuse nach distal bewegen sollte ist die Nadelschutzhülse in einer finalen Endposition im gesicherten Zustand weiter vorne als zuvor. An Stelle der Nadelschutzhülse kann auch die Schalthülse 19 oder eine weitere, mit der Nadelschutzhülse axialfest verbundene Hülse in ihren Endpositionen oder den Bewegungen dazwischen detektiert werden. In diesem Fall kann die Funktionsfähigkeit des Axialpositionsdetektors getestet werden durch Bewegen der Schalthülse in einem teilgefertigten Zustand des Autoinjektors, bevor eine Antriebseinheit mit der Schalthülse und dem Elektronikmodul und eine Spritzeneinheit mit der Nadelschutzhülse und der Spritze final zusammengefügt werden. Falls die Nadelschutzfeder, anders als in Fig.6 dargestellt, radial innerhalb der Schalthülse 19 angeordnet ist kann dadurch der Axialpositionssensor 24 noch weiter proximal platziert werden, insbesondere axial auf Höhe der in Fig.6 Mitte gezeigten vordersten Position des proximalen Endes der Schalthülse.

Die erstmalige Detektion der vorderen Endposition kann ein Einschalten, Aktivieren, und/oder Aufwecken von elektronischen Bauteilen des Elektronikmoduls bewirken, insbesondere des oben besprochenen Rotationssensors. Handelt es sich bei dem Axialpositionsdetektor um einen elektromechanischen Schalter kann durch Bewegen eines Kontaktelements, insbesondere durch Kippen eines Schalthebels hervorgerufen durch mechanischen Kontakt mit einer ersten Ausprägung auf der Nadelschutzhülse, ein elektrischer Kontakt erstmalig geschlossen werden. Dies wird in geeigneter Weise durch ein Prozessorelement in einem stromsparenden Tiefschlafmodus erkannt, in welchem das Prozessorelement auch während einer mehrjährigen Lagerdauer den Schalter überwacht. Durch das Prozessorelement werden anschliessend weitere Bauteile an die Energiespeichereinheit angeschlossen oder sonst wie aktiviert. Alternativ kann durch ein Kippen eines Schalthebels direkt zum Schliessen eines elektrischen Kontakts in einem Speisestromkreis genutzt werden, wodurch zuvor stromlose Komponenten des Elektronikmoduls eingeschaltet werden können. Weiter alternativ kann ein Speisestromkreis auch geschlossen werden durch Wegbewegen eines Isolators welcher zwei gegeneinander mechanisch vorgespannte Kontaktpunkte elektrisch trennt.

Fig.7 zeigt einen distalen Abschnitt der seitlichen Leiterplatine 21, und einen Axialpositionsdetektor umfassend einen elektromechanischen Schalter 24a in perspektivischer Ansicht. Der Schalter 24a ist so axial positioniert dass ein Hebel des Schalters durch zwei Ausprägungen 12a oder Rippen an einem Arm der Nadelschutzhülse 12 in den Endpositionen der Nadelschutzhülse in entgegengesetzte Richtungen gekippt wird. Die geschickte Platzierung der zwei Ausprägungen 12a mit einem Abstand entsprechend einem Hub der Nadelschutzhülse 12 zwischen den Endpositionen ermöglicht den Einsatz eines einzigen bidirektionalen Schalters zur Detektion von zwei Zuständen. Der Hebel des dargestellten Schalters kehrt bevorzugt nach einer ersten Auslösung oder Verkippung selbstständig in seine unbetätigte Position zurück und ist dadurch bereit für eine nächste Auslösung in einer der beiden Richtungen. Die Ausprägungen können auch an einer Schalthülse angebracht sein, insbesondere wenn diese axial fest mit der Nadelschutzfeder verschnappt ist.

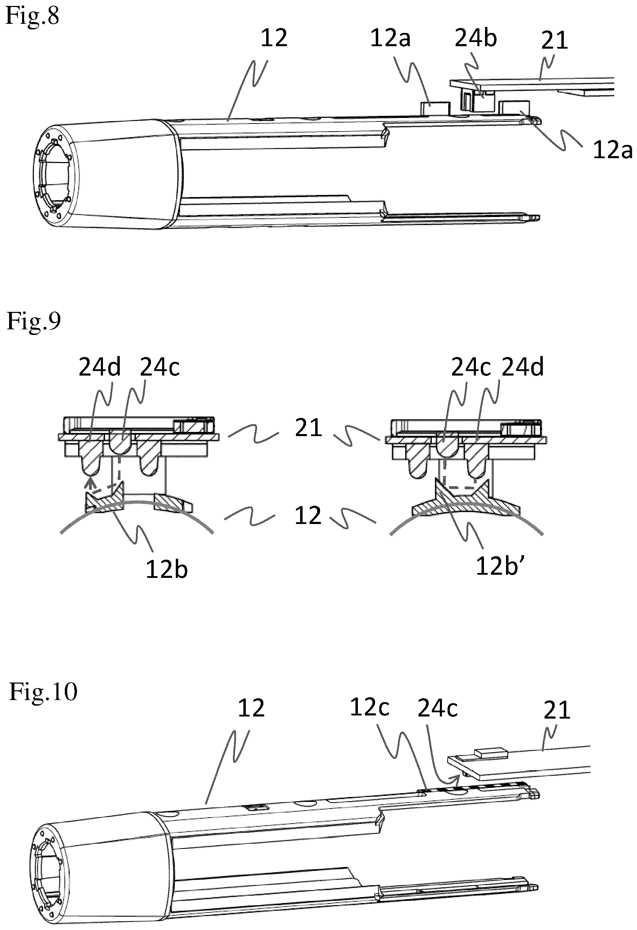

Fig.8 zeigt einen distalen Abschnitt der seitlichen Leiterplatine 21, und einen Axialpositionsdetektor umfassend eine Gabel-Lichtschranke 24b in perspektivischer Ansicht. Die Lichtschranke wird durch zwei Ausprägungen 12a oder Rippen an einem Arm der Nadelschutzhülse 12 in den Endpositionen der Nadelschutzhülse unterbrochen. Die Ausprägungen sind dazu in einem axialen Abstand entsprechend dem Hub der Nadelschutzhülse angeordnet. Eine Unterscheidung der beiden Endpositionen ist nur mit einer zweiten Lichtschranke möglich.

Fig.9 zeigt einen Schnitt durch den Autoinjektor auf Höhe eines Axialpositionsdetektors umfassend eine Reflexlichtschranke, mit der Nadelschutzhülse 12 in einer ersten (links) und in einer zweiten (rechts) Endposition der Nadelschutzhülse. Ein Lichtstrahl aus einer Lichtquelle 24c wird von zwei unterschiedlich ausgerichteten Spiegeln 12b, 12b` auf dem Arm der Nadelschutzhülse 12 in den Endpositionen zu zwei unterschiedlichen Lichtdetektoren 24d abgelenkt. Die Lichtquelle kann sichtbares Licht aussenden oder eine Infrarot-LED umfassen, jeder der Lichtdetektoren ist dann ein auf die Wellenlänge der Lichtquelle abgestimmter Foto-Transistor.

Fig. 10 zeigt einen distalen Abschnitt der seitlichen Leiterplatine 21, und einen Axialpositionsdetektor umfassend eine Reflexlichtschranke in perspektivischer Ansicht. Auf einer nach aussen gerichteten Seite eines Arms der Nadelschutzhülse 12 ist ein Muster 12c mit lichtabsorbierenden dunklen und lichtreflektierenden hellen Abschnitten aufgetragen. Die Reflexlichtschranke umfasst eine Lichtquelle 24c und einen Lichtdetektor zur Detektion des von der Lichtquelle emittierten und von einem hellen Abschnitt reflektierten Lichts. Alternativ oder zusätzlich zu einer unterschiedlichen Einfärbung der Abschnitte können diese auch eine abwechselnde Oberflächenbeschaffenheit aufweisen oder es können unterschiedlich geneigte Spiegelflächen aufgebracht werden, welche das Licht jeweils zum Detektor oder in eine andere Richtung lenken. Entsprechend einer axialen Ausdehnung der Abschnitte kann in dieser Variante die Distanz einer Verschiebung der Nadelschutzhülse mit einer gewünschten Auflösung gemessen werden, woraus die Einnahme einer Endposition identifiziert werden kann.

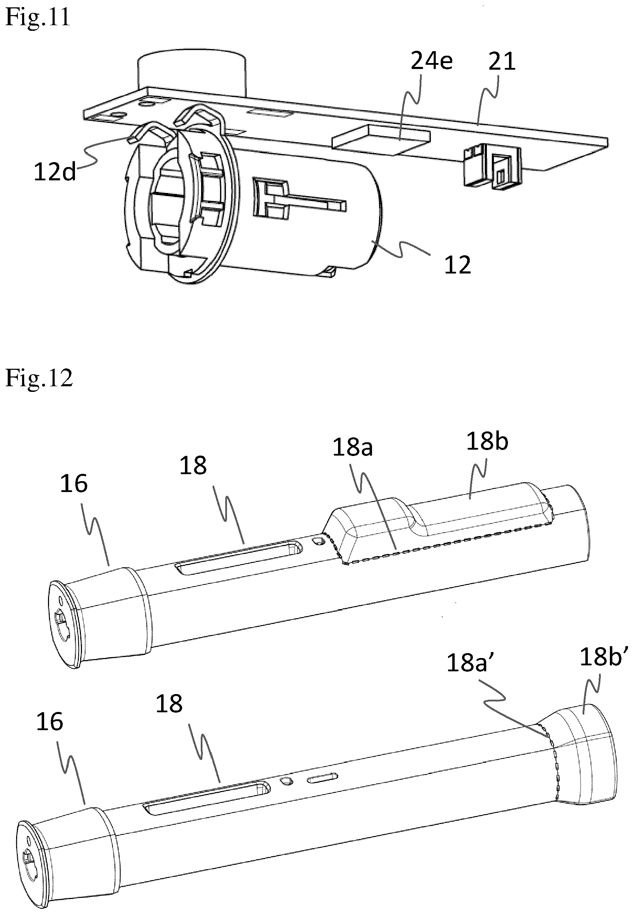

Fig. 11 zeigt einen Abschnitt der Nadelschutzhülse 12, eine seitliche Leiterplatine 21, und einen Axialpositionsdetektor umfassend einen Schleifkontaktsensor 24e in perspektivischer Ansicht. Der Schleifkontakt umfasst vorliegend ein axial fest mit der Nadelschutzhülse 12 verbundenes oder gekoppeltes Schleifelement 12d mit zwei flexiblen Armen, welche in bestimmten Axialpositionen Kontaktpunkte auf der Unterseite der Leiterplatine kontaktieren und zwischen diesen eine leitende Verbindung herstellen. Alternativ sind auch drei Schleifelemente denkbar, wovon eines permanent eine axiale Leiterbahn auf der Unterseite der Leiterplatine kontaktiert und die anderen beiden jeweils Abschnitte oder Punkte einer zweiten und einer dritten Leiterbahn kontaktieren. Die Abschnitte sind derart angeordnet dass abwechslungsweise zwischen der ersten und der zweiten Leiterbahn und zwischen der ersten und der dritten Leiterbahn ein detektierbarer elektrischer Kontakt besteht. Auch in dieser Variante kann die Distanz einer Verschiebung der Nadelschutzhülse mit einer Auflösung entsprechend der axialen Ausdehnung der Abschnitte gemessen werden, woraus die Einnahme einer Endposition identifiziert werden kann. Weiter alternativ wird durch den Schleifkontakt ein Widerstandselement mit einer axialen Ausdehnung entsprechend mindestens dem Hub der Nadelschutzhülse kontaktiert, entweder kontinuierlich oder in diskreten Schritten. Das Widerstandselement ist bevorzugt ebenfalls auf die Leiterplatine aufgedruckt und weist einen im Vergleich zu den übrigen Leiterbahnen hohen elektrischen Widerstand auf, so dass über den Schleifkontakt ein Messkreis mit einem messbar axialpositionsabhängigen Widerstand gebildet wird. Dadurch kann eine absolute Position von Schleifkontakt und Nadelschutzhülse bestimmt werden.

Fig. 12 zeigt eine perspektivische Ansicht zweier Autoinjektoren nach einem weiteren Aspekt der Erfindung. Das Gehäuse 18 der Autoinjektoren weist eine Trenn- oder Sollbruchlinie 18a, 18a' auf, entlang welcher ein peripherer Gehäuseteil 18b, 18b' vom Rest des Gehäuses irreversibel abgetrennt werden kann. Ein Elektronikmodul umfassend wahlweise eine der in Fig.1 gezeigten, seitlich oder proximal angeordneten Leiterplatinen mit zumindest der Prozessoreinheit und eine Energiespeichereinheit zu deren Speisung ist fest mit dem abtrennbaren Gehäuseteil 18b verbunden und höchstens über weitere Sollbruchstellen oder formschlüssige Kontaktstellen mit anderen Komponenten des Autoinjektors gekoppelt. Ein Abtrennen des besagten peripheren Gehäuseteils entfernt somit das Elektronikmodul vom Rest des Autoinjektors, wodurch das Elektronikmodul einer von den mechanischen und/oder medizinischen Komponenten des Autoinjektors getrennten Entsorgung zugeführt werden kann. Bevorzugt umfasst das Elektronikmodul alle entsorgungskritischen elektronischen Komponenten des erfindungsgemässen Autoinjektors, so dass keine elektronischen Bauteile nach Abtrennen des Elektronikmoduls im Autoinjektor zurückbleiben. Nicht als elektronische Komponenten im vorliegenden Sinne gelten über Schleif- oder Steckkontakte formschlüssig kontaktierbare elektronischen Leiterbahnen oder Drähte im Autoinjektor ausserhalb der Leiterplatine, wie beispielsweise das Schleifelement aus Fig.11, oder Aktoren basierend auf Shape-Memory Drähten. Die Elektronik umfasst dazu hauptsächlich Sensoren, welche selbst ohne Kraftaufwand von einem Gegenpart oder nicht-elektronischen Auslöseelement entfernt werden können. Die Sollbruchlinien im Gehäuse des Autoinjektors sind beispielsweise durch Perforation und/oder gezielte Schwächung der Gehäusedicke hervorgerufen, ebenso sind Halte- oder Griffpositionen vorgesehen, beispielsweise Rippen und/oder Noppen, so dass ein Nutzer ohne Einsatz von Werkzeugen nur mit Daumen und Finger einer Hand einen kleineren Teil des Gehäuses mitsamt der darin verankerten Leiterplatine von einem verbleibenden Teil des Gehäuses trennen und bestimmungsgemäss entsorgen kann. Beispielsweise kann eine am proximalen Ende vorgesehene Leiterplatine durch eine drehende oder schraubende Bewegung einer proximalen Endkappe gegenüber dem Gehäuse abgedreht werden wie dies vom Öffnen von versiegelten Getränkeflaschen bekannt ist.

Alternativ zu den Sollbruchlinien kann der abzutrennende periphere Gehäuseteil auch eine vom restlichen Gehäuse verschiedene, aber mit diesem lösbar verschnappte Komponente sein so dass nach Lösen der Verschnappung das periphere Gehäuseteil unmittelbar und ohne weitere Krafteinwirkung entfernt werden kann. Das Gehäuse weist dazu eine über die Kontur der Trennlinie in peripherer Richtung hinausgehende Verlängerung oder Erweiterung auf welche im verschnappten Zustand als Ganzes vom peripheren Gehäuseteil umgeben ist. Diese Gehäuseerweiterung trägt individuelle Schnappelemente welche von innen in Ausnehmungen im peripheren Gehäuseteil eingreifen. Es ist auch denkbar eine Trennung der Gehäuseteile nicht unmittelbar nach der Injektion durch den Patienten vornehmen zu lassen, sondern erst in einer Sammelstelle, wobei auch geeignete Werkzeuge zum Einsatz gelangen können. Weiter alternativ verbleibt das periphere Gehäuseteil nach öffnen der Trennlinie oder der Verschnappung über ein Scharnier mit dem restlichen Gehäuseteil verbunden, und das Elektronikmodul wird anschliessend unmittelbar separat ergriffen und entfernt. Durch eine von der Prozessoreinheit am Ende der Injektion oder mechanisch beim Entfernen des Autoinjektors von der Injektionsstelle freizugebende Verriegelung kann vermieden werden, dass die vorgeschlagene Trennung zu früh geschieht.

Die vorgenannten Trenn- oder Sollbruchlinien sind nicht zu verwechseln mit den Grenzen oder Konturen zwischen zwei Gehäuseteilen eines Autoinjektors, welcher zur einfacheren Montage aus zwei Untereinheiten oder Baugruppen zusammensetzbar ist. In diesem Fall umfasst eine distale Spritzeneinheit des Autoinjektors ein erstes, distales Gehäuseteil, die Nadelschutzhülse, die Gerätekappe und den Spritzenhalter, während eine proximale Antriebseinheit ein zweites, proximales Gehäuseteil, den Mechanikhalter, die Nadelschutzfeder, Sperr- und Schalthülse, Antriebs- und Vortriebselement, und den einmalig aufladbaren Energiespeicher für die automatische Substanzabgabe umfasst. Das proximale Gehäuseteil wird auch als Endkappe bezeichnet. In einem Abfüll- oder Montageprozess wird die Fertigspritze in die Spritzeneinheit eingeführt und die beiden Untereinheiten anschliessend zusammengeführt, wobei die beiden Gehäuseteile unlösbar verschnappen und an ihrer Oberfläche eine Grenzlinie bilden. Das vorgehend definierte Elektronikmodul ist bevorzugt Teil der Antriebseinheit, entsprechend umfasst das proximale Gehäuseteil die Trenn- oder Sollbruchlinie und das abtrennbare Gehäuseteil 18b vollständig.

Die Fertigspritze 10 umfasst einen zylindrischen Spritzenkörper als Produktbehälter, in welchem zwischen einer Spritzenschulter und einem entlang der Längsachse verschiebbaren Kolben oder Stopfen ein Produktaufnahmeraum begrenzt ist. An einem distalen Ende des Spritzenkörpers ist eine hohle Injektionsnadel mit der Spritzenschulter fest verbunden, und an einem proximalen Ende der Fertigspritze ist ein Fingerflansch angebracht, welcher radial nach aussen über den Aussenumfang des Spritzenkörpers ragt. Im Produktaufnahmeraum ist im Lieferzustand ein abzugebendes Produkt enthalten welches ganz oder teilweise durch Verschiebung des Stopfens von einer Stopfenstartposition in eine Stopfenendposition aus dem Produktaufnahmeraum durch die Injektionsnadel heraus verdrängt werden kann.

Die Injektionsnadel der Fertigspritze 10 ist von einer Nadelschutzkappe abgedeckt, welche als sogenanntes Rigid Needle Shield (RNS) ausgebildet ist und ein gummielastisches Nadelschutzelement und eine Hülle aus Hartkunststoff umfasst. Die Nadelschutzkappe schützt die Injektionsnadel gegen mechanische Einwirkungen und Verschmutzung, und hält die Injektionsnadel und das Produkt steril. An dem distalen Ende des Autoinjektors ist in seinem Lieferzustand eine Geräte- oder Abziehkappe 16 angeordnet, die vor der Verwendung des Autoinjektors axial abgezogen und/oder abgedreht und vollständig entfernt wird. Die Gerätekappe 16 weist ferner Schnapphaken auf, mit welchem die Nadelschutzkappe von der Fertigspritze gelöst wird. Der Spritzenhalter umfasst zwei elastische Finger, welche an ihren proximalen Enden an einer Halterhülse des Spritzenhalters befestigt sind und an ihren distalen Enden jeweils ein axiales Auflageelement für die Spritzenschulter aufweisen.

Die Injektionsnadel ist von einer relativ zum Gehäuse axial verschiebbar gelagerten und in das Gehäuse einschiebbaren Nadelschutzhülse 12 umgeben. In der Ausgangsposition der Nadelschutzhülse steht das distale Ende der Nadelschutzhülse distal über die Nadelspitze der Injektionsnadel über, so dass ein Zugriff auf die Nadelspitze zunächst verhindert wird. Die Nadelschutzhülse weist an ihrer distalen Stirnseite eine Öffnung auf, durch welche die Injektionsnadel bei einer Relativbewegung von Nadelschutzhülse und Injektionsnadel hindurch- und in eine Injektionsstelle eintreten kann. Die Nadelschutzhülse dient auch als Auslöseelement zum Auslösen der Produktausschüttung, wobei die Nadelschutzhülse hierzu unter Spannung einer Nadelschutzfeder 17 relativ zu dem Gehäuse in die proximale Richtung verschoben wird. Die Nadelschutzhülse umfasst dazu zwei Hülsenarme, welche gegenüber zwei als Sichtfenster bezeichneten Ausnehmungen des Gehäuses um 90° um die Längsachse versetzt beziehungsweise verdreht angeordnet sind. Nach der erfolgten Injektion kann die Nadelschutzhülse relativ zu dem Gehäuse aus der betätigten Position entlang der Längsachse in die distale Richtung in eine Nadelschutzposition verschoben und dort gegen erneutes Zurückschieben blockiert werden.

Der Autoinjektor umfasst ein Schaltmodul mit einer Schalthülse und einer von der Schalthülse umgebenen Sperrhülse. Die Schalthülse ist bevorzugt mit einem proximalen Ende der Hülsenarme der Nadelschutzhülse 12 verschnappt und wird von einem distalen Ende der Nadelschutzfeder 17 nach distal gedrängt. Die Nadelschutzfeder ist eine als Druckfeder wirkende und als Wendelfeder ausgestaltete Feder aus Metall. Die Sperrhülse ist dazu ausgebildet, die Schalthülse und die Nadelschutzhülse nach erfolgter Injektion in einer vorderen Endposition gegen erneutes Einschieben in das Gehäuse zu verriegeln. Nach radial aussen gerichtete Vorsprünge an den federnden Armen eines Verriegelungsglieds der Sperrhülse hintergreifen eine proximale Kante der Schalthülse, so dass sich die Schalthülse und damit die Nadelschutzhülse relativ zur Sperrhülse nicht in proximale Richtung bewegen können. Die Sperrhülse ist minimal beabstandet von einer nach distal gerichteten axialfesten Stirnfläche des Autoinjektors, so dass sich die Sperrhülse in proximaler Richtung höchstens um deutlich weniger als der Distanz zwischen Injektionsnadelspitze und distalem Ende der Nadelschutzfeder bewegen kann. Die Verriegelung wird erreicht durch einen proximalen Arretierhub der Sperrhülse relativ zur Schalthülse, bei welchem das Verriegelungsglied von der Schalthülse für eine Bewegung nach innen freigegeben wird und durch die Federwirkung der Arme eine nach proximal gerichtete Kante des Autoinjektors hintergreift. Beim anschliessenden Entfernen des Autoinjektors von der Einstichstelle wird die Schalthülse durch die Nadelschutzfeder in distaler Richtung über das Verriegelungsglied geschoben, worauf das Verriegelungsglied durch die Federwirkung der Arme in einer Verriegelungsposition eine nach proximal gerichtete Kante der Schalthülse hintergreift und die Schalthülse sowie die Nadelschutzhülse gegen eine erneute Bewegung in proximale Richtung blockiert. Eine korrekte Verriegelung der Nadelschutzhülse gegen erneutes Einschieben wird nicht separat detektiert sondern durch die beschriebene mechanische Ablaufsteuerung sichergestellt.

Das Elektronikmodul des Autoinjektors umfasst eine Leiterplatine und darauf angeordnet eine Sensoreinheit zur Detektion von Zuständen oder Vorgängen, eine Prozessoreinheit zur Verarbeitung von Signalen der Sensoreinheit, eine Kommunikationseinheit zur drahtlosen Kommunikation von Daten der Prozessoreinheit an ein Drittgerät, und eine Energiespeichereinheit zur Speisung der vorgenannten Einheiten. Die Energiespeichereinheit ist für eine Lagerdauer von mehreren Jahren mit minimalem Stromverbrauch und für einen einmaligen Einsatz des Autoinjektors mit kurzzeitiger Signalverarbeitung und Datenaustausch ausreichend aufgeladen. Eine als Energiespeichereinheit geeignete Batterie ist nicht wiederaufladbar aber bevorzugt in einem separaten Batteriefach für eine getrennte Entsorgung zugänglich. Die direkte Kommunikation mit einem stationären Drittgerät, beispielsweise einem Expertensystem in einer delokalisierten oder Cloud-basierten Infrastruktur, zur Übermittlung von Daten eines Injektionsprozesses kann beispielsweise über ein 5G- oder 4G/LTE Mobilfunknetzwerk, insbesondere ein Narrowband Intemet-of-Things NB-IoT, erfolgen, oder über ein anderes geeignetes Mittel wie LoRa, Sigfox, oder satellitengestützte Kommunikation. Zur Optimierung der geografischen Abdeckung bei moderater Erhöhung der Kosten und des Platzbedarfs können unterschiedliche Protokolle auf einem Dual-Mode Chip implementiert werden. Die Kommunikation mit einem mobilen Gerät, beispielsweise einem Mobiltelefon oder Smartphone, oder mit einem stationären Gateway zu einem drahtgebundenen Netzwerk, erfolgt bevorzugt über eine Bluetooth oder BLE Verbindung, welche vorteilhafterweise über ein Out-of-Band Pairing initiiert wird. Die BLE Kommunikation über das mobile Gerät des Anwenders kann zur Sicherstellung des Datenaustausches gleichzeitig oder redundant mit der direkten Kommunikation im Mobilfunknetzwerk geschehen. Die unterschiedlichen Kommunikationsmittel können auch modular vorgesehen sein, beispielsweise kann eine Mobilfunk-Kommunikationseinheit nachträglich eine BLE Kommunikationseinheit ersetzen oder ergänzen, bevorzugt zusammen mit einer angepassten Energiespeichereinheit. Zur Aufnahme und zur elektrischen Kontaktierung eines optionalen 5G Moduls mit zugehörigem Energiespeicher und passender SIM Karte können eine mechanische Halterung im Gerätegehäuse und ein Stecker mit Verbindung zur Leiterplatine standardmässig vorgesehen sein, so dass keine Komponenten des Autoinjektors in Abhängigkeit eines Entscheids für oder gegen ein 5G Modul angepasst werden müssen. Ohne 5G Modul bleiben diese Halterung und Stecker einfach ungenutzt. Die Sensoreinheit kann einen Temperatursensor umfassen zur Erfassung eines Temperaturverlaufs im Autoinjektor während einer Aufwärmphase, nachdem der Autoinjektor aus einem Kühlschank entnommen wurde. Eine Erwärmung der gekühlt gelagerten Medikamente vor der subkutanen Injektion kann die empfundenen Schmerzen verringern, entsprechend ist es hilfreich, wenn dem Nutzer das Erreichen einer Zieltemperatur signalisiert werden kann. Dabei muss der Temperatursensor nicht zwingend in oder auf der Fertigspritze montiert sein, da ein Temperaturverlauf an einer anderen Stelle, beispielsweise auf der Leiterplatine des Elektronikmoduls, für diese Zwecke ausreichend aussagekräftig ist. Insbesondere zeigt eine zeitliche Verzögerung der Medikamententemperatur gegenüber der Sensortemperatur ein reproduzierbares, vom Füllvolumen der Spritze und der Umgebungstemperatur unabhängiges Verhalten, und kann für eine bestimmte Sensorposition empirisch bestimmt werden. Im Falle eines nahezu konstanten zeitlichen Temperaturvorsprungs des Sensors kann ein Zieltemperaturzeitpunkt des Medikaments einfach abgeschätzt werden, indem bei Erreichen der Zieltemperatur am Sensor eine vorbestimmte Zeitspanne im Bereich von 30-90 Sekunden abgewartet wird, wobei der tiefere Wert für Sensorpositionen in unmittelbarer Nähe der Fertigspritze gilt. Alternativ kann bei annähernd exponentieller Annäherung der Sensortemperatur an eine unbekannte Umgebungstemperatur ein kritischer Temperaturgradient ermittelt werden, bei dessen Unterschreiten auf eine ausreichende Erwärmung des Medikaments geschlossen werden kann. Der Temperatursensor wird bevorzugt durch den Anwender bei Entnahme des Autoinjektors aus dem Kühlschrank aktiv eingeschaltet. Dies kann durch einen Schalter zum Schliessen eines elektrischen Kontakts in einem Speisestromkreis erfolgen, oder durch Wegbewegen eines Isolators welcher zwei gegeneinander mechanisch vorgespannte Kontaktpunkte elektrisch trennt, und/oder durch Entfernen einer Abrissfolie.

Die Temperatur des Autoinjektors beziehungsweise des Medikaments kann durch den Temperatursensor über die gesamte Transportkette überwacht und registriert werden. So kann beispielsweise sichergestellt werden, dass eine Maximaltemperatur während dem Transport inklusive Weg zum Kühlschrank des Anwenders nicht oder höchstens während einer akzeptablen Zeitdauer überschritten wurde. Zur ressourcenschonenden und präzisen Bestimmung eines kritischen Zeitpunktes mit Temperaturüberschreitung in der Lieferkette kann die Sensoreinheit einen geeigneten zeitstabilen Schwingquarz aufweisen als Frequenz- oder Taktgeber. Die Schwingungen des Quarzes werden gezählt für die Zeitspanne ab dem kritischen Zeitpunkt bis zu dem Moment in welchem die Prozessoreinheit aufgeweckt oder synchronisiert ist, und daraus rückwirkend der kritische Kalenderzeitpunkt bestimmt. Dank dem dezidierten Schwingquarz kann darauf verzichtet werden, einen Prozessor mit einer kalibrierten Uhr während der gesamten Transport- und Lagerdauer aktiv zu betreiben. Das Elektronikmodul umfasst weiter eine optische, akustische, und/oder taktile Anzeigeeinheit wie beispielsweise eine optische Anzeige, bei welcher ein Lichtleiter das Licht einer Lichtquelle auf der Leiterplatine an die Oberfläche des Gehäuses leitet. Ein durch die Anzeigeeinheit angezeigter Zustand kann einen Gerätezustand des Autoinjektors, einen Modulzustand des Elektronikmoduls, oder einen Vorgangszustand eines laufenden oder abgeschlossenen Injektionsvorganges umfassen. Die Anzeigeeinheit des Elektronikmoduls kann einfach gehalten werden und sich auf einige wenige LEDs, beispielsweise in Ampelfarben oder zur Beleuchtung ausgewählter Piktogramme, und/oder einen akustischen Signalgenerator zur Erzeugung von sprachunabhängigen Geräuschen oder Melodien beschränken. Dies ist insbesondere im Zusammenwirken mit den fortgeschrittenen graphischen Anzeigemöglichkeiten und Sprachausgabemöglichkeiten eines Smartphones vorteilhaft, da das drahtlos an das Elektronikmodul gekoppelte Smartphone die verfeinerte, über eine Statusanzeige hinausgehende Kommunikation mit dem Benutzer übernimmt. Alternativ dazu kann die Anzeigeeinheit Teil einer vollständigen Mensch-Maschine Schnittstelle sein, welche weiter über einen Bildschirm und/oder Sprachausgabemöglichkeiten zur Information eines Nutzers sowie über Eingabemöglichkeiten wie Schalter, kapazitive Berührungssensoren, und/oder Spracherkennung zur Ansteuerung der Einheiten verfügt.

Das mobile Gerät muss initial eingerichtet und konfiguriert werden, etwa durch installieren einer Applikation und Registrierung des Benutzers. Dies kann durch eine Patientendatenkarte geschehen, welche über Nahfeldradiokommunikation (NFC) oder optische QR Codes alle relevanten Daten auf das mobile Gerät überspielt. Falls das mobile Gerät sich in Reichweite des Autoinjektors befindet kann eine Übertragung von Injektionsdaten während einem Injektionsvorgang in Echtzeit erfolgen. In diesem Fall kann das mobile Gerät in Echtzeit Instruktionen an den Benutzer abgeben und diesen so durch die nächsten Schritte führen. Allenfalls können die Injektionsinformationen auch im Elektronikmodul gespeichert werden und erst später, in konsolidierter Form, übertragen werden. Die vom mobilen Gerät empfangenen Daten können durch den Benutzer ergänzt werden, etwa durch Angabe der Injektionsstelle, und werden auf geeignetem Weg an ein Expertensystem weitergegeben. Letzteres speichert die Daten und versorgt Patienten, medizinisches Personal, und Krankenversicherer mit gezielten Informationen und unterstützt so eine Befolgung eines Therapieplans durch den Benutzer des Injektionsgerätes.

### BEZUGSZEICHENLISTE

| | | | |
|---|---|---|---|
| 1 | Autoinjektor | 18 | Gehäuse |
| 10 | Fertigspritze | 18a | Trennlinie |
| 11 | Spritzenhalter | 18b | Gehäuseteil |
| 12 | Nadelschutzhülse | 19 | Schalthülse |
| 12a | Ausprägung | 20 | Elektronikmodul |
| 12b | Spiegel | 21 | Leiterplatine |
| 12c | Muster | 22 | Rotationssensor |
| 12d | Schleifelement | 22a | Gabellichtschranke |
| 13 | Antriebselement | 22b | Schalter |
| 14 | Vortriebselement | 22c | Hall Sensor |
| 15 | Torsionsfeder | 23 | Energiequelle |
| 15a | Federspule | 24 | Axialpositionsdetektor |
| 15b | Flansch | 24a | Schalter |
| 15c | Modulation | 24b | Gabellichtschranke |
| 15d | Muster | 24c | Lichtquelle |
| 15e | Permanentmagnet | 24d | Lichtdetektor |
| 16 | Gerätekappe | 24e | Schleifkontaktsensor |
| 17 | Nadelschutzfeder | | |

## Patentansprüche

1. Autoinjektor (1) mit einem Gehäuse (18), einer vorgefüllten Einweg-Fertigspritze (10) umfassend einen Produktbehälter und eine daran unlösbar befestigte Injektionsnadel, einer Torsionsfeder (15) zur einmaligen Ausschüttung eines maximalen Inhalts des Produktbehälter, einem Antriebselement (13), und einem Vortriebselement (14), wobei zur Ausschüttung die Torsionsfeder (15) das Antriebselement (13) in Rotation versetzt und das rotierende Antriebselement (13) eine Vortriebsbewegung des Vortriebselements (14) und eines Kolbens im Produktbehälter bewirkt,
wobei der Autoinjektor einen Rotationssensor (22) umfasst zur abwechslungsweisen kontinuierlichen Detektion von mindestens zwei Rotationspositionen pro Umdrehung des Antriebselementes (13) während der Ausschüttung, **dadurch gekennzeichnet, dass** der Autoinjektor (1) eine Prozessoreinheit zur Bestimmung einer axialen Kolbenposition des Kolbens im Produktbehälter aus den detektierten Rotationspositionen und eine Kommunikationseinheit zur Kommunikation mit einem Drittgerät oder eine Anzeigeeinheit zur Anzeige eines Zustandes des Autoinjektors umfasst, wobei die Prozessoreinheit, die Kommunikationseinheit oder die Anzeigeeinheit, und eine Energiequelle zur Speisung der Einheiten auf einer Leiterplatine (21) angeordnet sind, welche zusammen mit einem abtrennbaren Gehäuseteil (18b) des Autoinjektors (1) von einem Rest des Autoinjektors (1) entfernt werden kann.

2. Autoinjektor nach Anspruch 1, **dadurch gekennzeichnet, dass** das Vortriebselement (14) ein Axialführungselement aufweist für eine ausschliesslich lineare Vortriebsbewegung im Gehäuse.

3. Autoinjektor nach Anspruch 1 oder 2, **dadurch gekennzeichnet, dass** die Einweg-Fertigspritze (10) axial fixiert im Gehäuse (18) gehalten ist.

4. Autoinjektor nach Anspruch 1, **gekennzeichnet durch** eine Signalisierungseinheit, welche von der Prozessoreinheit nach Bestimmen einer axialen Kolbenposition entsprechend einer vollständigen Ausschüttung und nach anschliessendem Ablauf einer vorbestimmten Haltezeit zur Signalisierung eines Endes der Ausschüttung angesteuert wird.

5. Autoinjektor nach Anspruch 1, umfassend einen Axialpositionsdetektor (24) zur Detektion einer Sicherungsbewegung einer Nadelschutzhülse aus einer hinteren Endposition in eine vordere Endposition zur Abdeckung einer Injektionsnadel, **dadurch gekennzeichnet dass** die Prozessoreinheit ausgebildet ist, eine im Zeitpunkt der Detektion der Sicherungsbewegung bestimmte Kolbenposition oder eine entsprechende Flüssigkeitsmenge abzuspeichern.

6. Autoinjektor nach einem der Ansprüche 1 bis 5, **dadurch gekennzeichnet, dass** der Rotationssensor (22) umfasst einen unsymmetrisch ausgebildeten rotierbaren Aktor und ein durch den Aktor mechanisch auslenkbares oder optisch abdeckbares Abtastelement.

7. Autoinjektor nach einem der Ansprüche 1 bis 6, **dadurch gekennzeichnet, dass** der Rotationssensor (22) umfasst eine rotierbare Oberfläche mit unterschiedlich reflektierenden Sektoren, und einen Photodetektor zum Erfassen von sektoriell reflektiertem Licht.

8. Autoinjektor nach einem der Ansprüche 1 bis 7, **dadurch gekennzeichnet, dass** der Rotationssensor (22) umfasst einen elektromagnetischen Sensor und eine rotierbare Anordnung von passiven Aktoren.

9. Autoinjektor nachnach einem der Ansprüche 1 bis 8, **dadurch gekennzeichnet, dass** der abtrennbare Gehäuseteil durch vorgegebene Sollbruchlinien (18a) im Gehäuse des Autoinjektors definiert ist.

10. Autoinjektor nach einem der Ansprüche 1 bis 9, **dadurch gekennzeichnet, dass** das abtrennbare Gehäuseteil (18b) mittels einer drehenden oder schraubenden Bewegung vom Rest des Autoinjektors entfernt werden kann.

11. Autoinjektor nach einem der Ansprüche 1 bis 10, **dadurch gekennzeichnet, dass** eine mechanisch freizugebende Verriegelung die Trennung des abtrennbaren Gehäuseteils (18b) vor der Injektion verhindert.

12. Autoinjektor nach Anspruch 1, mit einer Gerätekappe (16), einer Nadelschutzhülse (12) und einer Nadelschutzfeder (17), wobei in einem Lieferzustand die Nadelschutzhülse durch die Nadelschutzfeder in einen ersten Anschlag gedrängt wird, und nach Entfernen der Nadelschutzkappe aber vor der Ausschüttung die Nadelschutzhülse durch die Nadelschutzfeder in einen zweiten Anschlag gedrängt wird, weiter umfassend einen Axialpositionsdetektor (24) zur Detektion einer Bewegung der Nadelschutzhülse in distale Richtung in den zweiten Anschlag.

13. Autoinjektor nach Anspruch 12, **dadurch gekennzeichnet dass** der Axialpositionsdetektor (24) in einer proximalen Hälfte des Autoinjektors angeordnet ist.

14. Autoinjektor nach Anspruch 12 oder 13, **dadurch gekennzeichnet dass** der Axialpositionsdetektor (24) eine Bewegung der Nadelschutzhülse aus einer hinteren Endposition, in welche die Nadelschutzhülse bei Kontakt und Anpressen an eine Injektionsstelle in proximale Richtung bewegt wird, in den zweiten Anschlag detektiert.

## Claims

1. Autoinjector (1) having a housing (18), a disposable pre-filled syringe (10) comprising a product container and an injection needle permanently attached thereto, a torsion spring (15) for one-time dispensing of a maximum content of the product container, a drive element (13), and a propulsion element (14), the torsion spring (15), for dispensing, setting the drive element (13) in rotation and the rotating drive element (13) causing a propulsion movement of the propulsion element (14) and a piston in the product container,
the autoinjector comprising a rotation sensor (22) for alternately and continuously detecting at least two rotational positions per revolution of the drive element (13) during dispensing, **characterized in that** the autoinjector (1) comprises a processor unit for determining an axial piston position of the piston in the product container from the detected rotational positions, and a communication unit for communicating with a third apparatus or a display unit for displaying a state of the autoinjector, the processor unit, the communication unit or the display unit, and an energy source for supplying the units being arranged on a printed circuit board (21) which, together with a detachable housing part (18b) of the autoinjector (1), can be removed from a remainder of the autoinjector (1).

2. Autoinjector according to claim 1, **characterized in that** the propulsion element (14) has an axial guide element for an exclusively linear propulsion movement in the housing.

3. Autoinjector according to claim 1 or 2, **characterized in that** the disposable pre-filled syringe (10) is held axially fixed in the housing (18).

4. Autoinjector according to claim 1, **characterized by** a signaling unit which is controlled by the processor unit after determining an axial piston position corresponding to complete dispensing and after a predetermined holding time has subsequently elapsed to signal an end of dispensing.

5. Autoinjector according to claim 1, comprising an axial position detector (24) for detecting a securing movement of a needle protection sleeve from a rear end position into a front end position for covering an injection needle, **characterized in that** the processor unit is designed to store a piston position determined at the time of detecting the securing movement or a corresponding amount of liquid.

6. Autoinjector according to any of claims 1 to 5, **characterized in that** the rotation sensor (22) comprises an asymmetrical, rotatable actuator and a scanning element which can be mechanically deflected or optically covered by the actuator.

7. Autoinjector according to any of claims 1 to 6, **characterized in that** the rotation sensor (22) comprises a rotatable surface having sectors which reflect differently, and a photodetector for detecting light reflected in the sectors.

8. Autoinjector according to any of claims 1 to 7, **characterized in that** the rotation sensor (22) comprises an electromagnetic sensor and a rotatable arrangement of passive actuators.

9. Autoinjector according to any of claims 1 to 8, **characterized in that** the separable housing part is defined by predetermined break lines (18a) in the housing of the autoinjector.

10. Autoinjector according to any of claims 1 to 9, **characterized in that** the separable housing part (18b) can be removed from the remainder of the autoinjector by means of a rotating or screwing movement.

11. Ainjector according to any of claims 1 to 10, **characterized in that** a lock which can be released mechanically prevents the detachable housing part (18b) from being separated before injection.

12. Autoinjector according to claim 1, having an apparatus cap (16), a needle protection sleeve (12) and a needle protection spring (17), wherein, in a delivery state, the needle protection sleeve is pushed into a first stop by the needle protection spring, and after removal of the needle protection cap but before dispensing of the needle protection sleeve, the needle protection sleeve is pushed into a second stop by the needle protection spring, the autoinjector further comprising an axial position detector (24) for detecting a movement of the needle protection sleeve in the distal direction.

13. Autoinjector according to claim 12, **characterized in that** the axial position detector (24) is arranged in a proximal half of the autoinjector.

14. Autoinjector according to claim 12 or 13, **characterized in that** the axial position detector (24) detects a movement of the needle protection sleeve from a rear end position, into which the needle protection sleeve is moved in the proximal direction when the sleeve contacts and presses against an injection site, into the second stop.

## Revendications

1. Auto-injecteur (1) comportant un boîtier (18), une seringue prête à l'emploi jetable (10) préremplie comprenant un réservoir de produit et une aiguille d'injection fixée de manière non amovible à celui-ci, un ressort de torsion (15) permettant la distribution unique d'un contenu maximal du réservoir de produit, un élément d'entraînement (13), et un élément d'avancement (14), pour la distribution, le ressort de torsion (15) mettant en rotation l'élément d'entraînement (13) et l'élément d'entraînement (13) en rotation provoquant un mouvement d'avancement de l'élément d'avancement (14) et d'un piston dans le réservoir de produit,
l'auto-injecteur comprenant un capteur de rotation (22) permettant de détecter alternativement en continu au moins deux positions de rotation par révolution de l'élément d'entraînement (13) pendant la distribution, **caractérisé en ce que** l'auto-injecteur (1) comprend une unité de traitement permettant de déterminer une position de piston axiale du piston dans le réservoir de produit à partir des positions de rotation détectées et une unité de communication permettant de communiquer avec un appareil tiers ou une unité d'affichage permettant d'afficher un état de l'auto-injecteur, l'unité de traitement, l'unité de communication ou l'unité d'affichage, et une source d'énergie permettant d'alimenter les unités, étant disposées sur une carte de circuit imprimé (21), laquelle carte de circuit imprimé peut être retirée d'un reste de l'auto-injecteur (1) conjointement avec une partie de boîtier séparable (18b) de l'auto-injecteur (1).

2. Auto-injecteur selon la revendication 1, **caractérisé en ce que** l'élément d'avancement (14) présente un élément de guidage axial pour un mouvement d'avancement uniquement linéaire dans le boîtier.

3. Auto-injecteur selon la revendication 1 ou 2, **caractérisé en ce que** la seringue prête à l'emploi jetable (10) est maintenue de manière fixe axialement dans le boîtier (18).

4. Auto-injecteur selon la revendication 1, **caractérisé par** une unité de signalisation qui est commandée par l'unité de traitement après la détermination d'une position de piston axiale en fonction d'une distribution complète et après l'écoulement ultérieur d'un temps de maintien prédéterminé pour la signalisation de la fin de la distribution.

5. Auto-injecteur selon la revendication 1, comprenant un détecteur de position axiale (24) permettant de détecter un mouvement de sécurisation d'un manchon de protection d'aiguille d'une position d'extrémité arrière vers une position d'extrémité avant pour le recouvrement d'une aiguille d'injection, **caractérisé en ce que** l'unité de traitement est configurée pour stocker une position de piston déterminée au moment de la détection du mouvement de sécurisation ou une quantité de liquide correspondante.

6. Auto-injecteur selon l'une des revendications 1 à 5, **caractérisé en ce que** le capteur de rotation (22) comprend un actionneur rotatif conçu de façon asymétrique et un élément de détection pouvant être dévié mécaniquement par l'actionneur ou pouvant être recouvert optiquement.

7. Auto-injecteur selon l'une des revendications 1 à 6, **caractérisé en ce que** le capteur de rotation (22) comprend une surface rotative comportant des secteurs à réflexions différentes, et un photodétecteur permettant de détecter une lumière réfléchie par secteurs.

8. Auto-injecteur selon l'une des revendications 1 à 7, **caractérisé en ce que** le capteur de rotation (22) comprend un capteur électromagnétique et un agencement rotatif d'actionneurs passifs.

9. Auto-injecteur selon l'une des revendications 1 à 8, **caractérisé en ce que** la partie de boîtier séparable est définie par des lignes de rupture de consigne (18a) prédéfinies dans le boîtier de l'auto-injecteur.

10. Auto-injecteur selon l'une des revendications 1 à 9, **caractérisé en ce que** la partie de boîtier séparable (18b) peut être retirée du reste de l'auto-injecteur à l'aide d'un mouvement de rotation ou de vissage.

11. Auto-injecteur selon l'une des revendications 1 à 10,
**caractérisé en ce qu'**un moyen de verrouillage à libérer mécaniquement empêche la séparation de la partie de boîtier séparable (18b) avant l'injection.

12. Auto-injecteur selon la revendication 1, comportant un capuchon d'appareil (16), un manchon de protection d'aiguille (12) et un ressort de protection d'aiguille (17), dans un état de fourniture, le manchon de protection d'aiguille étant poussé par le ressort de protection d'aiguille dans une première butée, et, après le retrait du capuchon de protection d'aiguille mais avant la distribution, le manchon de protection d'aiguille étant poussé par le ressort de protection d'aiguille dans une seconde butée, comprenant en outre un détecteur de position axiale (24) permettant de détecter un mouvement du manchon de protection d'aiguille dans la direction distale dans la seconde butée.

13. Auto-injecteur selon la revendication 12, **caractérisé en ce que** le détecteur de position axiale (24) est disposé dans une moitié proximale de l'auto-injecteur.

14. Auto-injecteur selon la revendication 12 ou 13, **caractérisé en ce que** le détecteur de position axiale (24) détecte un mouvement du manchon de protection d'aiguille d'une position d'extrémité arrière, dans laquelle le manchon de protection d'aiguille est déplacé dans la direction proximale lors du contact et de la pression contre un point d'injection, vers la seconde butée.
